(19) Europäisches Patentamt / European Patent Office / Office européen des brevets

(11) **EP 3 364 423 B1**

(12) **EUROPÄISCHE PATENTSCHRIFT**

(45) Veröffentlichungstag und Bekanntmachung des Hinweises auf die Patenterteilung:
**01.04.2020 Patentblatt 2020/14**

(21) Anmeldenummer: **17156490.9**

(22) Anmeldetag: **16.02.2017**

(51) Int Cl.:
*H01B 1/22* (2006.01)    *H01B 1/24* (2006.01)
*B32B 27/00* (2006.01)    *B33Y 70/00* (2020.01)
*B33Y 10/00* (2015.01)    *B29C 64/153* (2017.01)
*B29C 64/165* (2017.01)    *B29C 64/205* (2017.01)
*B29C 64/264* (2017.01)    *B33Y 30/00* (2015.01)

(54) **ELEKTRISCH LEITFÄHIGE BESCHICHTUNG MIT PARTIKELANTEILGRADIENTEN, INSBESONDERE FÜR MEDIZINISCHE GERÄTE**

ELECTRIC COATING WITH A PARTICLE GRADIENT, IN PARTICULAR FOR MEDICAL DEVICES

COUCHE ELECTRIQUE AVEC UN GRADIENT DE PARTICULE, EN PARTICULIER POUR DES APPAREILS MEDICAUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**22.08.2018 Patentblatt 2018/34**

(73) Patentinhaber: **Heraeus Deutschland GmbH & Co. KG**
**63450 Hanau (DE)**

(72) Erfinder:
- **Trötzschel, Jens**
 **63549 Ronneburg (DE)**
- **Gaiser, Detlef**
 **51061 Köln (DE)**
- **Schibli, Stefan**
 **60326 Frankfurt (DE)**

(74) Vertreter: **Herzog IP Patentanwalts GmbH**
**Immermannstraße 40**
**40210 Düsseldorf (DE)**

(56) Entgegenhaltungen:
WO-A2-2007/095058    WO-A2-2009/058453
WO-A2-2011/029058    DE-A1-102009 012 673
DE-A1-102010 013 038    US-A1- 2014 246 220
US-B1- 6 174 606

- **KODAMA H: "Automatic method for fabricating a three-dimensional plastic model with photo-hardening polymer", REVIEW OF SCIENTIFIC INSTRUMENTS, AIP, MELVILLE, NY, US, Bd. 52, Nr. 11, 1. November 1981 (1981-11-01), Seiten 1770-1773, XP002083706, ISSN: 0034-6748, DOI: 10.1063/1.1136492**

**Beschreibung**

Die vorliegende Erfindung betrifft einen Verbund, beinhaltend als einander überlagernde Schichten einer Schichtfolge

    a) ein Substrat, und
    b) eine erste Schicht;

wobei die erste Schicht

    i) eine erste Schichtoberfläche,
    ii) ein Polymer, und
    iii) eine Vielzahl elektrisch leitfähiger Partikel

beinhaltet; wobei die erste Schichtoberfläche an das Substrat angrenzt; wobei mindestens in einer ersten Region die erste Schicht in einem ersten Abstand von der ersten Schichtoberfläche gekennzeichnet ist durch einen ersten Anteil der elektrisch leitfähigen Partikel; wobei mindestens in der ersten Region die erste Schicht in einem weiteren Abstand von der ersten Schichtoberfläche gekennzeichnet ist durch einen weiteren Anteil der elektrisch leitfähigen Partikel; wobei der erste Anteil weniger ist als der weitere Anteil; wobei der erste Abstand weniger ist als der weitere Abstand. Weiter betrifft die Erfindung eine Vorrichtung, ein Verfahren, ein elektrisches Bauelement, ein elektrisches Gerät, und Verwendungen eines 3D-Druckers sowie einer Zusammensetzung.

In vielfältigen Anwendungen kommen elektrisch leitfähige Beschichtungen einiger $\mu$m Dicke auf einem Substrat zum Einsatz. Soll eine solche dünne elektrisch leitfähige Beschichtung elektrisch kontaktiert werden ergeben sich im Stand der Technik gravierende Probleme. Ist die elektrisch leitfähige Beschichtung eine metallische Beschichtung einiger $\mu$m Dicke, so neigt diese bei mechanischer Belastung zum Abblättern oder zur Rissbildung. Eine mechanische Belastung kann hierbei von einer (elastischen) Verformung des Substrats ausgehen oder von einer mechanischen Kontaktierung der Beschichtung selbst. Die eher brüchige metallische Beschichtung vermag somit nicht eine dauerhafte, leicht elektrisch kontaktierbare Beschichtung eines Substrats darzustellen. Alternativ sind im Stand der Technik elektrisch leitfähige Kunststoffe zur Bildung von Beschichtungen auf Substraten bekannt. Werden diese elektrisch leitfähigen Kunststoffbeschichtungen mechanisch kontaktiert, beispielsweise durch Klemmen oder Federn, besteht zu dem kontaktierenden Element ein beträchtlicher Kontaktwiderstand, welcher bei nur einigen $\mu$m dicken Schichten den elektrischen Widerstand der Schicht selbst übersteigen kann. Dieser Kontaktwiderstand kann im Stand der Technik nur durch Aufbringen von Kontaktlacken verringert werden. Somit weisen sowohl metallische als auch elektrisch leitfähige Kunststoffbeschichtungen erhebliche technische Nachteile auf bezüglich ihrer elektrischen Kontaktierung.

Diese elektrische Kontaktierung kann im Stand der Technik auf verschiedene Arten erfolgen. Das hierbei bekannte Schweißen oder Löten ist auf Grund der dabei auftretenden hohen Temperaturen nicht für temperaturempfindliche Substrate geeignet. Ferner können wenige $\mu$m dicke Beschichtungen kaum geschweißt oder gelötet werden. Ein elektrisches Kontaktieren mittels elektrisch leitfähiger Epoxidharze ist sehr aufwendig. Ferner ist die Kontaktierung hier irreversibel, also nicht wieder lösbar. Mechanische Kontaktierungen sind grundsätzliche wieder lösbar, führen jedoch wie oben beschrieben bei elektrisch leitfähigen Kunststoffbeschichtungen zu erheblichen Kontaktwiderständen. Letztlich ist im Stand der Technik das elektrische Kontaktieren durch Bonden bekannt. Diese Kontaktierungsart eignet sich grundsätzlich nur für metallische Beschichtungen, wobei die Beschichtung eine Mindestfläche aufweisen und zudem möglichst flach sein muss. US2014/246220A1 wird als nächster Stand der Technik angesehen, da darin einen ähnlicher Verbund offenbart wird.

Allgemein ist es eine Aufgabe der vorliegenden Erfindung, einen Nachteil, der sich aus dem Stand der Technik ergibt, zumindest teilweise zu überwinden. Eine weitere Aufgabe der Erfindung ist es, eine elektrisch leitfähige Beschichtung, überlagernd ein Substrat, bereitzustellen, wobei die Beschichtung eine möglichst vorteilhafte Kombination der folgenden Eigenschaften aufweist: eine hohe elektrische Leitfähigkeit, ein geringer Kontaktwiderstand, eine hohe Haltbarkeit, eine hohe mechanische Stabilität, insbesondere gegenüber elastischen Verformungen, eine hohe Haftfestigkeit und eine möglichst freie Strukturierbarkeit in 3 Dimensionen. Eine weitere Aufgabe der Erfindung ist es die vorgenannte Beschichtung bereitzustellen, wobei das Substrat aus einem Kunststoff, insbesondere einem Polymers, besteht. Eine weitere Aufgabe der Erfindung ist es die vorgenannte Beschichtung bereitzustellen, wobei die Beschichtung biokompatibel ist. Eine weitere Aufgabe der Erfindung ist es, ein elektrisches Gerät oder ein elektrisches Bauelement oder beides, beinhaltend die vorgenannte Beschichtung bereitzustellen. Ferner ist es eine Aufgabe der Erfindung, die vorgenannte Beschichtung bereitzustellen, wobei die Beschichtung ein mehrphasiger elektrischer Leiter ist. Eine weitere Aufgabe der Erfindung ist es, einen 3D-Drucker zum Erzeugen der vorgenannten Beschichtung bereitzustellen.

Ein Beitrag zur mindestens teilwesen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen.

Einen Beitrag zur Erfüllung mindestens einer der erfindungsgemäßen Aufgaben leistet eine Ausführungsform 1 eines Verbunds, beinhaltend als einander überlagernde Schichten einer Schichtfolge

a) ein Substrat, und
b) eine erste Schicht;

wobei die erste Schicht

i) eine erste Schichtoberfläche,
ii) ein Polymer, und
iii) eine Vielzahl elektrisch leitfähiger Partikel

beinhaltet;

wobei die erste Schichtoberfläche an das Substrat angrenzt; wobei mindestens in einer ersten Region die erste Schicht in einem ersten Abstand von der ersten Schichtoberfläche gekennzeichnet ist durch einen ersten Anteil der elektrisch leitfähigen Partikel; wobei mindestens in der ersten Region die erste Schicht in einem weiteren Abstand von der ersten Schichtoberfläche gekennzeichnet ist durch einen weiteren Anteil der elektrisch leitfähigen Partikel; wobei der erste Anteil weniger ist als der weitere Anteil; wobei der erste Abstand weniger ist als der weitere Abstand. Bevorzugt ist der erste Anteil um einen Wert in einem Bereich von 0,1 bis 95 % weniger als der weitere Anteil. Dabei ist der erste Abstand bevorzugt um einen Wert in einem Bereich von 0,05 bis 1000 $\mu$m weniger als der weitere Abstand. Bevorzugter ist der erste Anteil um einen Wert in einem Bereich von 10 bis 90 % weniger als der weitere Anteil. Dabei ist der erste Abstand bevorzugter um einen Wert in einem Bereich von 0,1 bis 500 $\mu$m weniger als der weitere Abstand. Am bevorzugtesten ist der erste Anteil um einen Wert in einem Bereich von 20 bis 80 % weniger als der weitere Anteil. Dabei ist der erste Abstand am bevorzugtesten um einen Wert in einem Bereich von 1 bis 250 $\mu$m weniger als der weitere Abstand.

Eine erfindungsgemäße Ausführungsform 2 des Verbunds ist nach der Ausführungsform 1 ausgestaltet, wobei mindestens in der ersten Region die erste Schicht in einem zweiten Abstand von der ersten Schichtoberfläche gekennzeichnet ist durch einen zweiten Anteil der elektrisch leitfähigen Partikel; wobei der zweite Anteil weniger ist als der weitere Anteil und mehr ist als der erste Anteil; wobei der zweite Abstand weniger ist als der weitere Abstand und mehr ist als der erste Abstand. Bevorzugt ist der zweite Anteil um mindestens 0,1 %, bevorzugter um mindestens 20 % weniger als der weitere Anteil und mehr als der erste Anteil. Der zweite Abstand ist bevorzugt um einen Wert in einem Bereich von 0,05 bis 1000 $\mu$m, bevorzugt von 0,1 bis 500 $\mu$m, bevorzugter von 1 bis 250 $\mu$m, weniger als der weitere Abstand und mehr als der erste Abstand.

Eine erfindungsgemäße Ausführungsform 3 des Verbunds ist nach der Ausführungsform 1 oder 2 ausgestaltet, wobei mindestens in der ersten Region die erste Schicht in jedem i-ten Abstand von der ersten Schichtoberfläche gekennzeichnet ist durch einen i-ten Anteil der elektrisch leitfähigen Partikel; wobei jeder i-te Anteil weniger ist als der (i+1)-te Anteil und mehr ist als der (i-1)-te Anteil; wobei der i-te Abstand weniger ist als der (i+1)-te Abstand und mehr ist als der (i-1)-te Abstand; wobei der n-te Anteil weniger ist als der weitere Anteil und mehr ist als der (n-1)-te Anteil; wobei der n-te Abstand weniger ist als der weitere Abstand und mehr ist als der (n-1)-te Abstand; wobei i ein Index in dem Bereich 2 bis (n-1) ist, wobei n eine natürliche Zahl größer 2 ist. Bevorzugt ist jeder i-te Anteil um 0,1 %, bevorzugter um mindestens 20 %, weniger als der (i+1)-te Anteil und mehr als der (i-1)-te Anteil. Der i-te Abstand ist bevorzugt um einen Wert in einem Bereich von 0,05 bis 1000 $\mu$m, bevorzugt von 0,1 bis 500 $\mu$m, bevorzugter von 1 bis 250 $\mu$m, weniger als der (i+1)-te Abstand und mehr als der (i-1)-te Abstand.

Eine erfindungsgemäße Ausführungsform 4 des Verbunds ist nach einer der vorherigen Ausführungsformen ausgestaltet, wobei die erste Schicht in der ersten Region dadurch gekennzeichnet ist, dass ein Anteil der elektrisch leitfähigen Partikel von dem ersten Abstand zu dem weiteren Abstand, bevorzugt monoton, zunimmt. Dies bedeutet, dass der Anteil der elektrisch leitfähigen Partikel von dem ersten Abstand zu dem weiteren Abstand bevorzugt auf keiner Teilstrecke abnimmt, jedoch auf einer oder mehreren Teilstrecken konstant bleiben kann. Bevorzugt nimmt der Anteil der elektrisch leitfähigen Partikel von dem ersten Abstand zu dem weiteren Abstand stufenweise oder kontinuierlich zu.

Eine erfindungsgemäße Ausführungsform 5 des Verbunds ist nach einer der vorherigen Ausführungsformen ausgestaltet, wobei die erste Schicht weiter eine der ersten Schichtoberfläche gegenüberliegende weitere Schichtoberfläche beinhaltet, wobei die erste Schicht in der ersten Region dadurch gekennzeichnet ist, dass ein Anteil der elektrisch leitfähigen Partikel entlang einer Geraden von der ersten Schichtoberfläche zu der weiteren Schichtoberfläche eine monoton steigende Funktion von einem Abstand von der ersten Schichtoberfläche ist. Hierbei ist die Funktion nicht notwendigerweise streng monoton steigend.

Eine erfindungsgemäße Ausführungsform 6 des Verbunds ist nach einer der vorherigen Ausführungsformen ausgestaltet, wobei die erste Schicht an der ersten Schichtoberfläche gekennzeichnet ist durch einen Anteil der elektrisch leitfähigen Partikel in einem Bereich von 0 bis 20 %, bevorzugt von 0 bis 10 %, bevorzugter von 0 bis 5 %, jeweils bezogen auf die erste Schichtoberfläche.

Eine erfindungsgemäße Ausführungsform 7 des Verbunds ist nach einer der vorherigen Ausführungsformen ausgestaltet, wobei die elektrisch leitfähigen Partikel aus einem ausgewählt aus der Gruppe bestehend aus Gold, Silber, Palladium, Platin, und Kohlenstoff, oder einer Kombination aus mindestens zwei davon bestehen.

Eine erfindungsgemäße Ausführungsform 8 des Verbunds ist nach einer der vorherigen Ausführungsformen ausgestaltet, wobei die elektrisch leitfähigen Partikel gekennzeichnet sind durch eine Länge in einem Bereich von 1 bis 1000 $\mu$m, bevorzugt von 5 bis 500 $\mu$m, bevorzugter von 10 bis 100 $\mu$m.

Eine erfindungsgemäße Ausführungsform 9 des Verbunds ist nach einer der Ausführungsformen 1 bis 8 ausgestaltet, wobei die elektrisch leitfähigen Partikel gekennzeichnet sind durch einen Durchmesser in einem Bereich von 0,1 bis 1000 nm, bevorzugt von 0,5 bis 500 nm, bevorzugter von 1 bis 100 nm.

Eine erfindungsgemäße Ausführungsform 10 des Verbunds ist nach einer der vorherigen Ausführungsformen ausgestaltet, wobei das Polymer eines ausgewählt aus der Gruppe bestehend aus Silikon, einem elektrisch leitfähigem Polymer, einem Lack, einem Polyaromaten, einem Thermoplast und einem Harz, oder eine Kombination aus mindestens zwei davon ist.

Eine erfindungsgemäße Ausführungsform 11 des Verbunds ist nach einer der vorherigen Ausführungsformen ausgestaltet, wobei der Verbund gemäß der hierin beschriebenen Prüfmethode biegsam ist.

Eine erfindungsgemäße Ausführungsform 12 des Verbunds ist nach einer der vorherigen Ausführungsformen ausgestaltet, wobei die erste Schicht gekennzeichnet ist durch

a) einen Kontaktwiderstand für einen elektrischen Kontakt zu dem Substrat in einem Bereich von 0,1 $\Omega$ bis 20 k$\Omega$, bevorzugt von 0,1 $\Omega$ bis 10 k$\Omega$, bevorzugter von 0,1 $\Omega$ bis 5 k$\Omega$, am bevorzugtesten von 0,1 $\Omega$ bis 1 k$\Omega$, oder
b) eine Gesamtdicke in einem Bereich von 1 bis 3000 $\mu$m, bevorzugt von 1 bis 2000 $\mu$m, bevorzugter von 1 bis 1500 $\mu$m, am bevorzugtesten von 1 bis 500 $\mu$m, oder
c) beides.

Eine erfindungsgemäße Ausführungsform 13 des Verbunds ist nach einer der vorherigen Ausführungsformen ausgestaltet, wobei der Verbund weiter eine zusätzliche Schicht beinhaltet, wobei die zusätzliche Schicht

a) die erste Schicht mindestens teilweise überlagert,
b) eine zusätzliche erste Schichtoberfläche beinhaltet,
c) das Polymer beinhaltet,
d) eine zusätzliche Vielzahl der elektrisch leitfähigen Partikel beinhaltet;

wobei die zusätzliche erste Schichtoberfläche an die erste Schicht angrenzt, wobei mindestens in einer weiteren Region der zusätzlichen Schicht die zusätzliche Schicht in einem zusätzlichen ersten Abstand von der zusätzlichen ersten Schichtoberfläche gekennzeichnet ist durch einen zusätzlichen ersten Anteil der elektrisch leitfähigen Partikel; wobei mindestens in der weiteren Region die zusätzliche Schicht in einem zusätzlichen weiteren Abstand von der zusätzlichen ersten Schichtoberfläche gekennzeichnet ist durch einen zusätzlichen weiteren Anteil der elektrisch leitfähigen Partikel; wobei der zusätzliche erste Anteil mehr ist als der zusätzliche weitere Anteil; wobei der zusätzliche erste Abstand weniger ist als der zusätzliche weitere Abstand. Bevorzugt ist der zusätzliche erste Anteil um einen Wert in einem Bereich von 0,1 bis 95 % mehr als der zusätzliche weitere Anteil. Dabei ist der zusätzliche erste Abstand bevorzugt um einen Wert in einem Bereich von 0,05 bis 1000 $\mu$m weniger als der weitere Abstand. Bevorzugter ist der zusätzliche erste Anteil um einen Wert in einem Bereich von 10 bis 90 % mehr als der zusätzliche weitere Anteil. Dabei ist der zusätzliche erste Abstand bevorzugter um einen Wert in einem Bereich von 0,1 bis 500 $\mu$m weniger als der zusätzliche weitere Abstand. Am bevorzugtesten ist der zusätzliche erste Anteil um einen Wert in einem Bereich von 20 bis 80 % mehr als der zusätzliche weitere Anteil. Dabei ist der zusätzliche erste Abstand am bevorzugtesten um einen Wert in einem Bereich von 1 bis 250 $\mu$m weniger als der zusätzliche weitere Abstand.

Eine erfindungsgemäße Ausführungsform 14 des Verbunds ist nach der Ausführungsform 13 ausgestaltet, wobei mindestens in der weiteren Region die zusätzliche Schicht in einem zusätzlichen zweiten Abstand von der zusätzlichen ersten Schichtoberfläche gekennzeichnet ist durch einen zusätzlichen zweiten Anteil der elektrisch leitfähigen Partikel; wobei der zusätzliche zweite Anteil mehr ist als der zusätzliche weitere Anteil und weniger ist als der zusätzliche erste Anteil; wobei der zusätzliche zweite Abstand weniger ist als der zusätzliche weitere Abstand und mehr ist als der zusätzliche erste Abstand. Bevorzugt ist der zusätzliche zweite Anteil um mindestens 0,1 %, bevorzugter um mindestens 20 % mehr als der zusätzliche weitere Anteil und weniger als der zusätzliche erste Anteil. Der zusätzliche zweite Abstand ist bevorzugt um einen Wert in einem Bereich von 0,05 bis 1000 $\mu$m, bevorzugt von 0,1 bis 500 $\mu$m, bevorzugter von 1 bis 250 $\mu$m, weniger als der zusätzliche weitere Abstand und mehr als der zusätzliche erste Abstand.

Eine erfindungsgemäße Ausführungsform 15 des Verbunds ist nach der Ausführungsform 13 oder 14 ausgestaltet, wobei mindestens in der weiteren Region die zusätzliche Schicht in jedem zusätzlichen j-ten Abstand von der zusätzlichen ersten Schichtoberfläche gekennzeichnet ist durch einen zusätzlichen j-ten Anteil der elektrisch leitfähigen Partikel; wobei jeder zusätzliche j-te Anteil mehr ist als der zusätzliche (j+1)-te Anteil und weniger ist als der zusätzliche (j-1)-te Anteil; wobei der zusätzliche j-te Abstand weniger ist als der zusätzliche (j+1)-te Abstand und mehr ist als der zusätzliche (j-1)-te Abstand; wobei der zusätzliche m-te Anteil mehr ist als der zusätzliche weitere Anteil und weniger ist als der zusätzliche (m-1)-te Anteil; wobei der zusätzliche m-te Abstand weniger ist als der zusätzliche weitere Abstand und mehr ist als der zusätzliche (m-1)-te Abstand; wobei j ein Index in dem Bereich 2 bis (m-1) ist, wobei m eine natürliche Zahl größer 2 ist. Bevorzugt ist jeder zusätzliche j-te Anteil um mindestens 0,1 %, bevorzugter um mindestens 20 %, mehr als der zusätzliche (j+1)-te Anteil und weniger als der zusätzliche (j-1)-te Anteil. Der zusätzliche j-te Abstand ist bevorzugt um einen Wert in einem Bereich von 0,05 bis 1000 $\mu$m weniger als der zusätzliche (j+1)-te Abstand und mehr als der zusätzliche (j-1)-te Abstand. Bevorzugt ist jeder zusätzliche m-te Anteil um mindestens 0,1 %, bevorzugter um mindestens 20 %, mehr als der zusätzliche weitere Anteil und weniger als der zusätzliche (m-1)-te Anteil. Der zusätzliche m-te Abstand ist bevorzugt um einen Wert in einem Bereich von 0,05 bis 1000 $\mu$m, bevorzugt von 0,1 bis 500 $\mu$m, bevorzugter von 1 bis 250 $\mu$m, weniger als der zusätzliche weitere Abstand und mehr als der zusätzliche (m-1)-te Abstand.

Eine erfindungsgemäße Ausführungsform 16 des Verbunds ist nach einer der Ausführungsformen 13 bis 15 ausgestaltet, wobei die zusätzliche Schicht mindestens in der weiteren Region dadurch gekennzeichnet ist, dass ein Anteil der elektrisch leitfähigen Partikel von dem zusätzlichen ersten Abstand zu dem zusätzlichen weiteren Abstand, bevorzugt monoton, abnimmt. Dies bedeutet, dass der Anteil der elektrisch leitfähigen Partikel von dem zusätzlichen ersten Abstand zu dem zusätzlichen weiteren Abstand bevorzugt auf keiner Teilstrecke zunimmt, jedoch auf einer oder mehreren Teilstrecken konstant bleiben kann. Bevorzugt nimmt der Anteil der elektrisch leitfähigen Partikel von dem ersten Abstand zu dem weiteren Abstand stufenweise oder kontinuierlich ab.

Eine erfindungsgemäße Ausführungsform 17 des Verbunds ist nach einer der Ausführungsformen 13 bis 16 ausgestaltet, wobei die zusätzliche Schicht weiter eine der zusätzlichen ersten Schichtoberfläche gegenüberliegende zusätzliche weitere Schichtoberfläche beinhaltet, wobei die zusätzliche Schicht mindestens in der weiteren Region dadurch gekennzeichnet ist, dass ein Anteil der elektrisch leitfähigen Partikel entlang einer Geraden von der zusätzlichen ersten Schichtoberfläche zu der zusätzlichen weiteren Schichtoberfläche eine monoton fallende Funktion von einem Abstand von der zusätzlichen ersten Schichtoberfläche ist. Hierbei ist die Funktion nicht notwendigerweise streng monoton fallend.

Eine erfindungsgemäße Ausführungsform 18 des Verbunds ist nach einer der Ausführungsformen 13 bis 17 ausgestaltet, wobei die zusätzliche Schicht weiter eine der zusätzlichen ersten Schichtoberfläche gegenüberliegende zusätzliche weitere Schichtoberfläche beinhaltet, wobei die zusätzliche Schicht an der zusätzlichen weiteren Schichtoberfläche gekennzeichnet ist durch einen Anteil der elektrisch leitfähigen Partikel in einem Bereich von 0 bis 20 %, bevorzugt von 0 bis 10 %, bevorzugter von 0 bis 5 %, jeweils bezogen auf die zusätzliche weitere Schichtoberfläche.

Eine erfindungsgemäße Ausführungsform 19 des Verbunds ist nach einer der Ausführungsformen 13 bis 18 ausgestaltet, wobei die zusätzliche Schicht weiter eine der zusätzlichen ersten Schichtoberfläche gegenüberliegende zusätzliche weitere Schichtoberfläche beinhaltet, wobei die zusätzliche Schicht an der zusätzlichen weiteren Schichtoberfläche eine spezifische elektrische Leitfähigkeit von weniger als 6500 S/m, bevorzugt von weniger als 6000 S/m, bevorzugter von weniger als 5500 S/m, am bevorzugtesten von weniger als 5000 S/m, hat. Bevorzugt ist die zusätzliche Schicht an der zusätzlichen weiteren Schichtoberfläche elektrisch isolierend.

Eine erfindungsgemäße Ausführungsform 20 des Verbunds ist nach einer der Ausführungsformen 13 bis 19 ausgestaltet, wobei die elektrisch leitfähigen Partikel der zusätzlichen Vielzahl elektrisch leitfähiger Partikel aus einem ausgewählt aus der Gruppe bestehend aus Gold, Silber, Palladium, Platin, und Kohlenstoff, oder einer Kombination aus mindestens zwei davon bestehen.

Eine erfindungsgemäße Ausführungsform 21 des Verbunds ist nach einer der Ausführungsformen 13 bis 20 ausgestaltet, wobei die elektrisch leitfähigen Partikel der zusätzlichen Vielzahl elektrisch leitfähiger Partikel gekennzeichnet sind durch eine Länge in einem Bereich von 1 bis 1000 $\mu$m, bevorzugt von 5 bis 500 $\mu$m, bevorzugter von 10 bis 100 $\mu$m.

Eine erfindungsgemäße Ausführungsform 22 des Verbunds ist nach einer der Ausführungsformen 13 bis 21 ausgestaltet, wobei die elektrisch leitfähigen Partikel der zusätzlichen Vielzahl elektrisch leitfähiger Partikel gekennzeichnet sind durch einen Durchmesser in einem Bereich von 0,1 bis 1000 nm, bevorzugt von 0,5 bis 500 nm, bevorzugter von 1 bis 100 nm.

Eine erfindungsgemäße Ausführungsform 23 des Verbunds ist nach einer der Ausführungsformen 13 bis 22 ausgestaltet, wobei die zusätzliche Schicht die erste Schicht in einem ersten Teilbereich der ersten Schicht überlagert, wobei eine Kontaktierungsschicht die erste Schicht in mindestens einem weiteren Teilbereich der ersten Schicht überlagert, wobei die Kontaktierungsschicht auf einer der ersten Schicht abgewandten Oberfläche der Kontaktierungsschicht gekennzeichnet ist durch einen Kontaktwiderstand in einem Bereich von 0,1 $\Omega$ bis 20 k$\Omega$, bevorzugt von 0,1 $\Omega$ bis 10 k$\Omega$, bevorzugter von 0,1 $\Omega$ bis 5 k$\Omega$, am bevorzugtesten von 0,1 $\Omega$ bis 1 k$\Omega$. Bevorzugt überlagert die Kontaktierungsschicht die erste Schicht in 2 voneinander durch die zweite Schicht getrennten Teilbereichen. Somit bildet die Kontaktierungsschicht bevorzugt mindestens 2 Elektroden durch die ein elektrischer Kontakt hergestellt werden kann. Eine bevorzugte Kontaktierungsschicht bildet einen elektrischen Kontakt. Bevorzugt bildet die Kontaktierungsschicht 2 voneinander durch die zusätzliche Schicht getrennte elektrische Kontakte. Die Kontaktierungsschicht beinhaltet bevorzugt die elektrisch leitfähigen Partikel zu einem Anteil in einem Bereich von 1 bis 100 %, bevorzugter von 1 bis 50 %, bevorzugter von 1 bis 30 %, bevorzugter von 5 bis 30 %, weiter bevorzugt von 5 bis 25 %, jeweils bezogen auf die der ersten Schicht abgewandten Oberfläche der Kontaktierungsschicht. Ferner beinhaltet die Kontaktierungsschicht bevorzugt das Polymer.

Eine erfindungsgemäße Ausführungsform 24 des Verbunds ist nach einer der Ausführungsformen 13 bis 23 ausgestaltet, wobei der erste Teilbereich an den weiteren Teilbereich angrenzt.

Eine erfindungsgemäße Ausführungsform 25 des Verbunds ist nach einer der vorhergehenden Ausführungsformen ausgestaltet, wobei das Substrat aus einem ausgewählt aus der Gruppe bestehend aus einem Kunststoff, einer Kunststoffmischung und einem Metall, oder aus einer Kombination von mindestens zwei davon besteht.

Eine erfindungsgemäße Ausführungsform 26 des Verbunds ist nach einer der vorhergehenden Ausführungsformen ausgestaltet, wobei das Substrat von einem ausgewählt aus der Gruppe bestehend aus einem medizinischen Gerät, einem medizinischen Hilfsmittel und einem elektrischen Gerät oder von einer Kombination aus mindestens zwei davon beinhaltet ist.

Eine erfindungsgemäße Ausführungsform 27 des Verbunds ist nach einer der vorhergehenden Ausführungsformen ausgestaltet, wobei das Substrat eines ausgewählt aus der Gruppe bestehend aus einem Schlauch, einem Katheter, einem Draht, einer Nadel, einer Sonde, einem Implantat, einer Folie, einer Kanüle und einem Lead, oder eine Kombination aus mindestens zwei davon ist.

Eine erfindungsgemäße Ausführungsform 28 des Verbunds ist nach einer der vorhergehenden Ausführungsformen ausgestaltet, wobei der Verbund eines ausgewählt aus der Gruppe bestehend aus einem medizinischen Gerät, einem medizinischen Hilfsmittel, einem Stecker und einer Buchse oder eine Kombination aus mindestens zwei davon ist.

Einen Beitrag zur Erfüllung mindestens einer der erfindungsgemäßen Aufgaben leistet eine Ausführungsform 1 einer Vorrichtung 1, beinhaltend ein Substrat und eine Beschichtung, wobei das Substrat eine Substratoberfläche beinhaltet;

wobei die Beschichtung

a) eine erste Oberfläche und eine weitere Oberfläche beinhaltet,
wobei die erste Oberfläche an die Substratoberfläche angrenzt,
wobei die weitere Oberfläche von dem Substrat abgewandt ist;
b) ein Polymer und eine Vielzahl elektrisch leitfähiger Partikel beinhaltet;
c) an der ersten Oberfläche gekennzeichnet ist durch einen Anteil der elektrisch leitfähigen Partikel in einem Bereich von 0 bis 20 %, bevorzugt von 0 bis 10 %, bevorzugter von 0 bis 5 %, jeweils bezogen auf die erste Oberfläche;
d) ein erstes Teilvolumen beinhaltet; und
e) in dem ersten Teilvolumen gekennzeichnet ist durch einen Anteil der elektrisch leitfähigen Partikel in einem Bereich von 1 bis 100 Vol.-%, bevorzugt von 1 bis 50 Vol.-%, bevorzugter von 1 bis 30 Vol.-%, bevorzugter von 5 bis 30 vol.-%, weiter bevorzugt von 5 bis 25 vol.-%, jeweils bezogen auf das Volumen der Beschichtung in dem ersten Teilvolumen;

wobei in einer ersten Region der Beschichtung

i) die Beschichtung dadurch gekennzeichnet ist, dass entlang einer Geraden von der ersten Oberfläche zu der weiteren Oberfläche ein Anteil der elektrisch leitfähigen Partikel in der Beschichtung eine Funktion von einer Position auf der Geraden mit mindestens einem ersten lokalen Maximum ist,
wobei das erste lokale Maximum von dem ersten Teilvolumen beinhaltet ist,
wobei die Funktion von dem ersten lokalen Maximum zu jeweils einem in Richtung der ersten Oberfläche und einem in Richtung der weiteren Oberfläche angrenzenden Minimum kontinuierlich oder in mindestens 2 Stufen, bevorzugt in mindestens 3 Stufen, bevorzugter in mindestens 4 Stufen, am bevorzugtesten in mindestens 5 Stufen, abnimmt; und
ii) die Beschichtung an der weiteren Oberfläche gekennzeichnet ist durch einen Anteil der elektrisch leitfähigen Partikel in einem Bereich von 0 bis 20 %, bevorzugt von 0 bis 10 %, bevorzugter von 0 bis 5 %, jeweils bezogen auf die weitere Oberfläche.

Eine erfindungsgemäße Ausführungsform 2 der Vorrichtung 1 ist nach der Ausführungsform 1 ausgestaltet, wobei in der ersten Region das erste Teilvolumen längserstreckt oder flächenförmig ist. Ein Volumen ist längserstreckt, wenn es in einer Dimension um mindestens einen Faktor 2, bevorzugt um mindestens einen Faktor 3, bevorzugter um mindestens einen Faktor 10, mehr ausgedehnt ist als in den beiden weiteren Dimensionen jeweils senkrecht zur ersten Dimension. Ein Volumen ist flächenförmig, wenn es in 2 Dimensionen jeweils um mindestens einen Faktor 2, bevorzugt um mindestens einen Faktor 3, bevorzugter um mindestens einen Faktor 10, mehr ausgedehnt ist als in der einen weiteren Dimension senkrecht zu jeder der beiden Dimensionen. Ein bevorzugtes flächenförmiges Volumen ist gekrümmt, bevorzugt zylindermantelflächenförmig gekrümmt.

Eine erfindungsgemäße Ausführungsform 3 der Vorrichtung 1 ist nach der Ausführungsform 1 oder 2 ausgestaltet, wobei die Beschichtung eine weitere Region beinhaltet, wobei das erste Teilvolumen in der weiteren Region die weitere Oberfläche der Beschichtung beinhaltet. Bevorzugt ist die weitere Oberfläche in der weiteren Region eine Kontaktierungsfläche mit einem Kontaktwiderstand in einem Bereich von 0,1 Ω bis 20 kΩ, bevorzugt von 0,1 Ω bis 10 kΩ, bevorzugter von 0,1 Ω bis 5 kΩ, am bevorzugtesten von 0,1 Ω bis 1 kΩ. In dieser Ausführungsform kann somit das erste Teilvolumen von außerhalb der Beschichtung an dem Teil der weiteren Oberfläche, der von dem ersten Teilvolumen beinhaltet ist, elektrisch kontaktiert werden. Demnach kann das erste Teilvolumen als elektrischer Leiter in der Beschichtung fungieren, zu dem über die weitere Oberfläche in der weiteren Region ein elektrischer Kontakt hergestellt werden kann.

Eine erfindungsgemäße Ausführungsform 4 der Vorrichtung 1 ist nach einer der Ausführungsformen 1 bis 3 ausgestaltet, wobei die Beschichtung

a) mindestens ein weiteres Teilvolumen beinhaltet,
b) in dem weiteren Teilvolumen gekennzeichnet ist durch einen Anteil der elektrisch leitfähigen Partikel in einem Bereich von 1 bis 100 Vol.-%, bevorzugt von 1 bis 50 Vol.-%, bevorzugter von 1 bis 30 Vol.-%, bevorzugter von 5 bis 30 vol.-%, weiter bevorzugt von 5 bis 25 Vol.-%, jeweils bezogen auf das Volumen der Beschichtung in dem weiteren Teilvolumen;

wobei in der ersten Region der Beschichtung die Funktion mindestens ein weiteres lokales Maximum beinhaltet, wobei das weitere lokale Maximum von dem weiteren Teilvolumen beinhaltet ist, wobei die Funktion von dem weiteren lokalen Maximum zu jeweils einem in Richtung der ersten Oberfläche und einem in Richtung der weiteren Oberfläche angrenzenden Minimum kontinuierlich oder in mindestens 2 Stufen, bevorzugt in mindestens 3 Stufen, bevorzugter in mindestens 4 Stufen, am bevorzugtesten in mindestens 5 Stufen, abnimmt, wobei das weitere Teilvolumen von dem ersten Teilvolumen elektrisch isoliert ist. Bevorzugt bilden das erste Teilvolumen und das weitere Teilvolumen jeweils eine Phase eines mehrphasigen elektrischen Leiters.

Eine erfindungsgemäße Ausführungsform 5 der Vorrichtung 1 ist nach einer der Ausführungsformen 1 bis 4 ausgestaltet, wobei das erste Teilvolumen oder das weitere Teilvolumen oder beide jeweils eine Elektrode ist. Eine bevorzugte Elektrode ist eine Ringelektrode.

Eine erfindungsgemäße Ausführungsform 6 der Vorrichtung 1 ist nach einer der Ausführungsformen 1 bis 5 ausgestaltet, wobei das Substrat mit der Beschichtung gemäß der hierin beschriebenen Prüfmethode biegsam ist.

Eine erfindungsgemäße Ausführungsform 7 der Vorrichtung 1 ist nach einer der Ausführungsformen 1 bis 6 ausgestaltet, wobei das Substrat von einem ausgewählt aus der Gruppe bestehend aus einem medizinischen Gerät, einem medizinischen Hilfsmittel und einem elektrischen Gerät oder von einer Kombination aus mindestens zwei davon beinhaltet ist.

Eine erfindungsgemäße Ausführungsform 8 der Vorrichtung 1 ist nach einer der Ausführungsformen 1 bis 7 ausgestaltet, wobei das Substrat eines ausgewählt aus der Gruppe bestehend aus einem Schlauch, einem Katheter, einem Draht, einer Nadel, einer Sonde, einem Implantat, einer Folie, einer Kanüle und einem Lead, oder eine Kombination aus mindestens zwei davon ist.

Eine erfindungsgemäße Ausführungsform 9 der Vorrichtung 1 ist nach einer der Ausführungsformen 1 bis 8 ausgestaltet, wobei die Vorrichtung eines ausgewählt aus der Gruppe bestehend aus einem medizinischen Gerät, einem medizinischen Hilfsmittel, einem Stecker und einer Buchse oder eine Kombination aus mindestens zwei davon ist.

Eine erfindungsgemäße Ausführungsform 10 der Vorrichtung 1 ist nach einer der Ausführungsformen 1 bis 9 ausgestaltet, wobei die elektrisch leitfähigen Partikel aus einem ausgewählt aus der Gruppe bestehend aus Gold, Silber, Palladium, Platin, und Kohlenstoff, oder einer Kombination aus mindestens zwei davon bestehen.

Eine erfindungsgemäße Ausführungsform 11 der Vorrichtung 1 ist nach einer der Ausführungsformen 1 bis 10 ausgestaltet, wobei die elektrisch leitfähigen Partikel gekennzeichnet sind durch eine Länge in einem Bereich von 1 bis 1000 µm, bevorzugt von 5 bis 500 µm, bevorzugter von 10 bis 100 µm.

Eine erfindungsgemäße Ausführungsform 12 der Vorrichtung 1 ist nach einer der Ausführungsformen 1 bis 11 ausgestaltet, wobei die elektrisch leitfähigen Partikel gekennzeichnet sind durch einen Durchmesser in einem Bereich von 0,1 bis 1000 nm, bevorzugt von 0,5 bis 500 nm, bevorzugter von 1 bis 100 nm.

Eine erfindungsgemäße Ausführungsform 13 der Vorrichtung 1 ist nach einer der Ausführungsformen 1 bis 12 ausgestaltet, wobei das Polymer eines ausgewählt aus der Gruppe bestehend aus Silikon, einem elektrisch leitfähigem Polymer, einem Lack, einem Polyaromaten, einem Thermoplast und einem Harz, oder eine Kombination aus mindestens zwei davon ist.

Eine erfindungsgemäße Ausführungsform 14 der Vorrichtung 1 ist nach einer der Ausführungsformen 1 bis 13 ausgestaltet, wobei die Beschichtung gekennzeichnet ist durch

a) einen Kontaktwiderstand zwischen der Substratoberfläche und der ersten Oberfläche in einem Bereich von 0,1 $\Omega$ bis 20 k$\Omega$, bevorzugt von 0,1 $\Omega$ bis 10 k$\Omega$, bevorzugter von 0,1 $\Omega$ bis 5 k$\Omega$, am bevorzugtesten von 0,1 $\Omega$ bis 1 k$\Omega$, oder
b) eine Gesamtdicke in einem Bereich von 8 $\mu$m bis 1 cm, bevorzugt von 10 $\mu$m bis 5 mm, bevorzugter von 50 $\mu$m bis 1 mm, oder
c) beides.

Einen Beitrag zur Erfüllung mindestens einer der erfindungsgemäßen Aufgaben leistet eine Ausführungsform 1 eines Verfahrens, beinhaltend als Verfahrensschritte

a) Bereitstellen eines Substrats und n Zusammensetzungen;
wobei das Substrat eine Substratoberfläche beinhaltet,
wobei jede der n Zusammensetzungen ein Polymer und eine Vielzahl von elektrisch leitfähigen Partikeln zu einem Partikelanteil, bezogen auf das Gewicht der jeweiligen Zusammensetzung, beinhaltet,
wobei die n Zusammensetzungen dadurch gekennzeichnet sind, dass sich die Partikelanteile der n Zusammensetzungen jeweils voneinander unterscheiden; und
b) Überlagern der Substratoberfläche mit jeweils mindestens einer ersten Portion der n Zusammensetzungen,

wobei das Überlagern mit mindestens den ersten Portionen der n Zusammensetzungen nacheinander erfolgt, wobei nach jedem Überlagern mit mindestens der ersten Portion einer der n Zusammensetzungen, die mindestens eine erste Portion gehärtet wird, wobei mindestens die ersten Portionen der n Zusammensetzungen in einer Reihenfolge zunehmender Partikelanteile überlagert werden, wobei eine erste Schichtfolge beinhaltend als sich einander überlagernde Schichten von der Substratoberfläche in einer Schichtfolgenrichtung eine erste bis n-te Schicht erhalten wird, wobei n eine natürliche Zahl größer 1, bevorzugt größer 2, bevorzugter größer 3, bevorzugter größer 4, am bevorzugtesten größer 5, ist. Gemäß Verfahrensschritt b) kann die Substratoberfläche jeweils mit mehreren Portionen einer der n Zusammensetzungen direkt nacheinander, das heißt ohne eine Portion einer Zusammensetzung mit einem anderen Partikelanteil dazwischen, überlagert werden. Dies ist insbesondere vorteilhaft, um eine bestimmte Schichtdicke zu erzeugen.
Eine erfindungsgemäße Ausführungsform 2 des Verfahrens ist nach der Ausführungsform 1 ausgestaltet, wobei das Verfahren weiter beinhaltet
c) Überlagern der n-ten Schicht der ersten Schichtfolge mindestens in einem Teilbereich der n-ten Schicht mit jeweils mindestens einer weiteren Portion der (n-1)-ten bis ersten Zusammensetzung,
wobei das Überlagern mit mindestens den weiteren Portionen der (n-1) Zusammensetzungen nacheinander erfolgt, wobei nach jedem Überlagern mit mindestens der weiteren Portion einer der (n-1) Zusammensetzungen, die mindestens eine weitere Portion gehärtet wird, wobei mindestens die weiteren Portionen der (n-1) Zusammensetzungen in einer Reihenfolge abnehmender Partikelanteile überlagert werden, wobei eine zweite Schichtfolge beinhaltend als sich einander überlagernde Schichten von der n-ten Schicht in der Schichtfolgenrichtung eine weitere (n-1)-te bis eine weitere erste Schicht erhalten wird. Gemäß Verfahrensschritt c) kann die n-te Schicht der ersten Schichtfolge jeweils mit mehreren weiteren Portionen einer der (n-1)-ten bis ersten Zusammensetzung direkt nacheinander, das heißt ohne eine weitere Portion einer Zusammensetzung mit einem anderen Partikelanteil dazwischen, überlagert werden. Dies ist insbesondere vorteilhaft, um eine bestimmte Schichtdicke zu erzeugen.
Eine erfindungsgemäße Ausführungsform 3 des Verfahrens ist nach der Ausführungsform 2 ausgestaltet, wobei in einem weiteren Verfahrensschritt d) eine Oberfläche der weiteren ersten Schicht elektrisch deaktiviert wird. Das vorgenannte elektrische Deaktivieren erfolgt bevorzugt, wenn das Polymer ein elektrisch leitfähiges Polymer, besonders bevorzugt PEDOT, ist. Bevorzugt wird die Oberfläche der weiteren ersten Schicht durch ein Ätzen elektrisch deaktiviert.
Eine erfindungsgemäße Ausführungsform 4 des Verfahrens ist nach einer der Ausführungsformen 1 bis 3 ausgestaltet, wobei das Härten der ersten Portionen oder das Härten der weiteren Portionen oder beides durch ein Bestrahlen mit Licht oder ein Erwärmen oder beides erfolgt. Ein bevorzugtes Bestrahlen mit Licht ist ein Bestrahlen mit Infrarotlicht oder ultraviolettem Licht oder beidem.
Eine erfindungsgemäße Ausführungsform 5 des Verfahrens ist nach der Ausführungsform 4 ausgestaltet, wobei das Erwärmen auf eine Temperatur in einem Bereich von 50 bis 300°C, bevorzugt von 50 bis 250°C, bevorzugter von 70 bis 200°C, bevorzugter von 80 bis 150°C, erfolgt.
Eine erfindungsgemäße Ausführungsform 6 des Verfahrens ist nach einer der Ausführungsformen 1 bis 5 ausgestaltet, wobei das Verfahren ein additives Fertigungsverfahren ist. Ein bevorzugtes additives Fertigungsverfahren ist ein 3D-Drucken. Zu unterscheiden ist das additive Fertigungsverfahren von subtraktiven Fertigungsverfahren. Ein weiteres bevorzugtes additives Fertigungsverfahren ist eines ausgewählt aus der Gruppe bestehend aus einem Pulverbettverfahren, einem Freiraumverfahren und einem Flüssigmaterialverfahren oder eine Kombination aus mindestens zwei davon. Ein besonders bevorzugtes Freiraumverfahren ist das Fused Desposition Modeling (FDM).

Eine erfindungsgemäße Ausführungsform 7 des Verfahrens ist nach einer der Ausführungsformen 1 bis 6 ausgestaltet, wobei das Verfahren mit einem 3D-Drucker durchgeführt wird.

Eine erfindungsgemäße Ausführungsform 8 des Verfahrens ist nach einer der Ausführungsformen 1 bis 7 ausgestaltet, wobei das Überlagern in Verfahrensschritt b) oder c) oder in beiden durch ein Auftragen mit einer Düse erfolgt, wobei die Düse eine Düsenöffnung mit einem Durchmesser in einem Bereich von 100 nm bis 2000 $\mu$m, bevorzugt von 200 nm bis 1000 $\mu$m, bevorzugter von 300 nm bis 500 $\mu$m, beinhaltet.

Eine erfindungsgemäße Ausführungsform 9 des Verfahrens ist nach einer der Ausführungsformen 1 bis 6 ausgestaltet, wobei das Überlagern in Verfahrensschritt b) oder c) oder in beiden durch ein Eintauchen erfolgt. Hierbei erfolgt das Eintauchen in Verfahrensschritt b) bevorzugt jeweils in eine der n Zusammensetzungen. Hierbei wird bevorzugt zunächst die Substratoberfläche eingetaucht und mit einer Portion einer Zusammensetzung benetzt. Die Portion wird gehärtet und somit eine erste Schicht erhalten. Eine Oberfläche der ersten Schicht wird in dieselbe oder eine andere Zusammensetzung mit größerem Partikelanteil eingetaucht und somit mit einer Portion der Zusammensetzung benetzt. Diese Portion wird wiederum gehärtet und somit eine zweite Schicht erhalten. Auf diese Weise werden die erste bis n-te Schicht erhalten. In Verfahrensschritt c) erfolgt das Eintauchen bevorzugt jeweils in eine der (n-1)-ten bis ersten Zusammensetzung.

Einen Beitrag zur Erfüllung mindestens einer der erfindungsgemäßen Aufgaben leistet eine Ausführungsform 1 einer Vorrichtung 2, erhältlich durch das Verfahren nach einer der Ausführungsformen 1 bis 9.

Einen Beitrag zur Erfüllung mindestens einer der erfindungsgemäßen Aufgaben leistet eine Ausführungsform 1 eines elektrischen Bauelements, beinhaltend den Verbund nach einer seiner Ausführungsformen 1 bis 28; oder die Vorrichtung 1 nach einer ihrer Ausführungsformen 1 bis 14; oder die Vorrichtung 2 nach ihrer Ausführungsform 1.

Einen Beitrag zur Erfüllung mindestens einer der erfindungsgemäßen Aufgaben leistet eine Ausführungsform 1 eines elektrischen Geräts, beinhaltend den Verbund nach einer seiner Ausführungsformen 1 bis 28; oder die Vorrichtung 1 nach einer ihrer Ausführungsformen 1 bis 14; oder die Vorrichtung 2 nach ihrer Ausführungsform 1; oder das elektrische Bauelement seiner Ausführungsform 1.

Eine erfindungsgemäße Ausführungsform 2 des elektrischen Geräts ist nach seiner Ausführungsformen 1 ausgestaltet, wobei das elektrische Gerät weiter einen Sensor beinhaltet.

Einen Beitrag zur Erfüllung mindestens einer der erfindungsgemäßen Aufgaben leistet eine Ausführungsform 1 einer Verwendung eines 3D-Druckers zum Erzeugen des Verbunds nach einer seiner Ausführungsformen 1 bis 28; oder zum Erzeugen der Vorrichtung 1 nach einer ihrer Ausführungsformen 1 bis 14; oder zum Erzeugen der Vorrichtung 2 nach ihrer Ausführungsform 1. Der 3D-Drucker beinhaltet bevorzugt eine Düse mit einer Düsenöffnung mit einem Durchmesser in einem Bereich von 100 nm bis 2000 $\mu$m, bevorzugt von 200 nm bis 1000 $\mu$m, bevorzugter von 300 nm bis 500 $\mu$m, beinhaltet.

Einen Beitrag zur Erfüllung mindestens einer der erfindungsgemäßen Aufgaben leistet eine Ausführungsform 1 einer Verwendung einer Zusammensetzung, beinhaltend ein Polymer und eine Vielzahl elektrisch leitfähiger Partikel, zu einem elektrischen Kontaktieren einer ein Substrat überlagernden Beschichtung unter Erhalt des erfindungsgemäßen Verbunds nach einer seiner Ausführungsformen.

Bevorzugte Ausgestaltungen von Bestandteilen einer erfindungsgemäßen Kategorie, insbesondere des Verbunds, der Vorrichtung und des Verfahrens, sind ebenso bevorzugt für gleichnamige oder entsprechende Bestandteile der anderen erfindungsgemäßen Kategorien.

In diesem Dokument bedeutet die Aussage, dass eine Entität eine andere überlagert, dass die Entität auf die andere Entität folgt. Hierbei kann die Entität mittelbar oder unmittelbar auf die andere Entität folgen. Demnach können die beiden Entitäten gemäß dieser Ausdrucksweise miteinander kontaktiert sein oder nicht. Dagegen bedeutet der Ausdruck, dass eine Entität an eine andere Entität angrenzt, dass die beiden Entitäten unmittelbar miteinander kontaktiert sind. Hierbei ist eine Entität typischerweise eine Schicht, ein Substrat oder eine Region.

Polymer

Ein bevorzugtes Polymer ist ein elektrisch leitfähiges Polymer. Ein bevorzugtes elektrisch leitfähiges Polymer ist Polyanilin oder ein Polyoxythiophen oder beides. Ein bevorzugtes Polyoxythiophen ist Poly-3,4-ethylendioxythiophen (PEDOT). Ein bevorzugtes PEDOT ist Poly-3,4-ethylendioxythiophen-Polystyrolsulfonat (PEDOT:PSS). Ein bevorzugtes elektrisch leitfähiges Polymer ist gekennzeichnet durch eine geringere spezifische elektrische Leitfähigkeit als die Partikel der Vielzahl der elektrisch leitfähigen Partikel, vorzugsweise um mindestens $1 \cdot 10^6$ S/m, bevorzugter um mindestens $10 \cdot 10^6$ S/m, am bevorzugtesten um mindestens $50 \cdot 10^6$ S/m.

Ein anderes bevorzugtes Polymer ist ein elektrisch nicht leitendes Polymer. Hierbei kommen insbesondere duroplastische, thermoplastische, und elastische Polymere in Betracht. Duroplastische Polymere schmelzen sehr schwer und - sofern diese überhaupt schmelzen - bei einer Temperatur von über 250°C Ein bevorzugtes duroplastisches Polymer ist ein Harz, insbesondere ein Epoxidharz, ein Polyaromat, vorzugsweise Parylen, oder Polyurethanharz, wobei ein Polyaromat, vorzugsweise Parylen, besonders bevorzugt ist. Ein bevorzugter Thermoplast ist einer ausgewählt aus der Gruppe bestehend aus einem Polycarbonat (PC), einem Polyethylen (PE), und Polymethylmethacrylat (PMMA), oder eine Kombination aus mindestens zwei davon. Ein bevorzugtes Polyethylen ist Polyterafluorethylen (PTFE). Ein bevorzugtes elastisches Polymer ist ein Latex, ein Gummi und besonders bevorzugt auf Silikon-basis. Das Polymer kann auch als Lack vorliegen. Ein bevorzugter Lack ist ein Fotolack. Ein bevorzugter Fotolack ist ein Negativresist oder ein Posistivresist oder beides. Ein bevorzugter Negativresist ist SU-8.

### Region

Im Kontext des erfindungsgemäßen Verbunds bezeichnet eine Region einer Schicht bevorzugt einen lateral flächigen Ausschnitt der Schicht in ihrer gesamten Dicke. Bevorzugt überlagert die weitere Region der zusätzlichen Schicht mindestens teilweise die erste Region der ersten Schicht.

### elektrisch leitfähige Partikel

Bevorzugte elektrisch leitfähige Partikel beinhalten das Polymer nicht. Weitere bevorzugte elektrisch leitfähige Partikel sind Metallpartikel oder Kohlenstoffpartikel oder beides. Bevorzugte Kohlenstoffpartikel sind Kohlenstoffröhrchen, bevorzugt Kohlenstoffnanoröhrchen. Bevorzugte Metallpartikel sind Drähte, bevorzugt Silber- oder Golddrähte. Weitere bevorzugte elektrisch leitfähige Partikel sind eines ausgewählt aus der Gruppe bestehend aus sphärisch, plättchenförmig, stäbchenförmig, röhrchenförmig, würfelförmig, quaderförmig und nadelförmig, oder eine Kombination aus mindestens zwei davon. Weitere bevorzugte elektrisch leitfähige Partikel sind längserstreckt. Bevorzugte längserstreckte elektrisch leitfähige Partikel sind eines ausgewählt aus der Gruppe bestehend aus röhrenförmig, prismenförmig, stäbchenförmig, drahtförmig, und fadenförmig, oder eine Kombination aus mindestens zwei davon. Weitere bevorzugte Kohlenstoffpartikel beinhalten, bevorzugter bestehen aus, Graphen oder Ruß (Carbon Black) oder beides.

### Substrat

Ein bevorzugter Kunststoff, aus dem das Substrat bevorzugt besteht, ist ein nicht leitendes Polymer. Dieses ist vorzugsweise ausgewählt aus der Gruppe bestehend aus Silikon, einem Polyaromaten, einem Thermoplast und einem Harz, oder eine Kombination aus mindestens zwei davon. Ein bevorzugter Polyaromat ist ein Parylen. Ein bevorzugtes Harz ist ein Epoxidharz. Ein bevorzugter Thermoplast ist einer ausgewählt aus der Gruppe bestehend aus einem Polycarbonat (PC), einem Polyethylen (PE), und Polymethylmethacrylat (PMMA), oder eine Kombination aus mindestens zwei davon. Ein bevorzugtes Polyethylen ist Polyterafluorethylen (PTFE). Besonders bevorzugt für ein Substrat sind ein Poylurethan, ein Polyaromat, insbesondere Parylen, ein Silikon, oder eine Kombination aus mindestens zwei davon. Eine bevorzugte Substratoberfläche ist eben oder gekrümmt oder beides. Eine bevorzugte gekrümmte Substratoberfläche ist mindestens teilweise eine Zylindermantelfläche.

### Zusammensetzung

Eine bevorzugte Zusammensetzung ist eine Suspension oder eine Paste oder beides. Eine bevorzugte Paste ist gekennzeichnet durch eine Viskosität in einem Bereich von 0,001 bis $10^8$ Pa·s, bevorzugter von 0,001 bis $10^7$ Pa·s, am bevorzugtesten von 0,001 bis $10^6$ Pa·s. Die Suspension oder Paste beinhaltet weiterhin ein Verdünnungsmittel. Dieses ist so gewählt, dass es bei einer Temperatur in einem Bereich von 40 bis 500°C, vorzugsweise in einem Bereich von 50 bis 200°C und besonders in einem Bereich von 60 bis 150°C verdampft. Besonders bevorzugte Verdünnungsmittel sind Tenside. Diese können ionisch oder nicht-ionisch vorliegen, wobei nicht-ionische Tenside besonders bevorzugt sind. Nicht-ionische Tenside sind beispielsweise unter dem Handelsnamen Tween® oder Triton® kommerziell erhältlich. Die Menge des Verdünnungsmittels wird in Abhängigkeit der weiteren Bestandteile der Zusammensetzung so gewählt, dass die jeweils gewünschte Viskosität der Zusammensetzung eingestellt wird. Eine bevorzugte Zusammensetzung beinhaltet neben dem Polymer und der Vielzahl elektrisch leitfähiger Partikeln weiterhin Verdünnungsmittel. Hierbei beinhaltet die Zusammensetzung mit dem geringsten Anteil elektrisch leitfähiger Partikel diese bevorzugt zu einem Anteil in einem Bereich von 0 bis 60 Gew.-%, bevorzugter von 0 bis 50 Gew.-%, am bevorzugtesten von 0 bis 40 Gew.-%, jeweils bezogen auf das Gewicht der Zusammensetzung. Ferner beinhaltet die Zusammensetzung mit dem größten Anteil elektrisch leitfähiger Partikel diese bevorzugt zu einem Anteil in einem Bereich von 20 bis 95 Gew.-%, bevorzugter von 30 bis 90 Gew.-%, am bevorzugtesten von 40 bis 85 Gew.-%, jeweils bezogen auf das Gewicht der Zusammensetzung.

elektrisches Deaktivieren

Ein bevorzugtes elektrisches Deaktivieren erfolgt durch ein Kontaktieren mit einem Halogen oder einer halogenhaltigen Verbindung oder beides. Bevorzugt wird die elektrisch zu deaktivierende Oberfläche hierbei partiell halogeniert.

medizinisches Gerät

Ein medizinisches Gerät ist ausgebildet zum Durchführen einer Therapie einer Krankheit oder einer Fehlfunktion; oder eines Diagnostikverfahrens; oder beides. Ein bevorzugtes medizinisches Gerät wird elektrisch betrieben. Ein weiteres bevorzugtes medizinisches Gerät ist ein Implantat. Ein bevorzugtes Implantat ist eines ausgewählt aus der Gruppe bestehend aus einem Schrittmacher, einem Biomonitor und einem Neurostimulator oder eine Kombination aus mindestens zwei davon. Hierbei ist das Substrat besonders bevorzugt von einem Lead des Schrittmachers beinhaltet ist. Ferner beinhaltet ein Schrittmacher einen elektrischen Impulsgeber.

medizinisches Hilfsmittel

Ein medizinisches Hilfsmittel ist ausgebildet, um bei einer medizinischen Behandlung eines menschlichen oder tierischen Körpers eingesetzt zu werden. Eine bevorzugte medizinische Behandlung ist hierbei eines ausgewählt aus der Gruppe bestehend aus einer Therapie, einem Eingriff und einer diagnostischen Untersuchung. Ein bevorzugter Eingriff beinhaltet ein Entnehmen aus dem menschlichen oder tierischen Körper oder ein Einbringen in den menschlichen oder tierischen Körper. Ein bevorzugtes Entnehmen ist ein Entnehmen einer Probe. Eine bevorzugte Probe ist eine Blutprobe oder eine Gewebeprobe. Ein bevorzugtes Einbringen ist ein Implantieren. Ein weiterer bevorzugter Eingriff ist ein chirurgischer Eingriff. Ein bevorzugtes medizinisches Hilfsmittel ist eines ausgewählt aus der Gruppe bestehend aus einer Nadel, einer Kanüle, einem Katheter und einem Beutel oder eine Kombination aus mindestens zwei davon. Ein bevorzugter Beutel ist ein Tropfbeutel oder ein Blutbeutel oder beides.

elektrisches Bauelement

Ein elektrisches Bauelement ist der grundlegende, als Einheit betrachtete Bestandteil einer elektrischen Schaltung. Ein bevorzugtes elektrisches Bauelement ist eines ausgewählt aus der Gruppe bestehend aus einem passiven Bauelement, einem aktiven Bauelement, einem linearen Bauelement, einem nichtlinearen Bauelement, einem diskreten Bauelement und einem integrierten Bauelement oder eine Kombination aus mindestens zwei davon. Ein bevorzugtes passives Bauelement ist eines ausgewählt aus der Gruppe bestehend aus einem Widerstand, einer Induktivität und einer Kapazität oder eine Kombination aus mindestens zwei davon. Ein bevorzugtes aktives Bauelement ist eine Transistor oder ein Relais oder beides.

elektrisches Gerät

Ein elektrisches Gerät ist ein durch elektrische Energie betriebenes Gerät für private oder gewerbliche Nutzung. Mit Hilfe von Elektrizität können damit eine oder mehrere Aufgaben erledigt werden. Das elektrische Gerätwird ist eines ausgewählt aus der Gruppe bestehend aus direkt vom Stromnetz mit Energie versorgbar, mit mindestens einem Akkumulator ausgestattet und mit mindestens einer Batterie ausgestattet oder eine Kombination aus mindestens zwei davon. Das elektrische Gerät beinhaltet eines oder mehrere elektrische Bauelemente.

MESSMETHODEN

Die folgenden Messmethoden wurden im Rahmen der Erfindung benutzt. Sofern nichts anderes angegeben ist wurden die Messungen bei einer Umgebungstemperatur von 25°C, einem Umgebungsluftdruck von 100 kPa (0,986 atm) und einer relativen Luftfeuchtigkeit von 50 % durchgeführt.

Partikelanteil

Zunächst wird mit einem Skalpell ein Querschnitt durch die Schichtfolge erzeugt. Hierbei wird der Querschnitt in dem Abstand zur Oberfläche der Schichtfolge erzeugt, in dem der Partikelanteil bestimmt werden soll. Somit wird eine Fläche der Schichtfolge offengelegt, die auf ihren Partikelanteil hin zu untersuchen ist. Hierbei wird der Abstand der Messposition von der Schichtoberfläche bzw. vom Substrat an dem Querschnitt mit einem Lichtmikroskop (mit Objektiv EPIPLAN 10 von Carl Zeiss Microscopy GmbH)überprüft. An der offengelegten Schnittfläche findet die Bestimmung des Partikelanteils statt.

Zunächst wird mit einem Rasterelektronenmikroskop (REM) ein Bild der zu untersuchenden Oberfläche aufgenommen. Hierzu wird das REM im Sekundärelektronenkontrastmodus (SE) mit einer Beschleunigungsspannung von 8 kV betrieben. Das Bild wird mit 10000-facher Vergrößerung erzeugt und als TIF-Datei gespeichert. In dem Bild sind die elektrisch leitfähigen Partikel gegenüber dem Polymer, in welchem sie eingebettet sind, heller dargestellt. Die REM-Aufnahme wird mit der Software ImageJ (Open Source, Version 1.51) geöffnet. Mit dem Cursor wird in dem Bild ein quadratischer Ausschnitt von 400 Pixeln × 400 Pixeln ausgewählt. Das Bild wird auf diesen Ausschnitt zugeschnitten und der Ausschnitt für die weitere Berabeitung geöffnet. Dies erfolgt durch die Befehle:

File - Edit - Cut;
File - New - Image mit den Standardeinstellungen Type: 8-bit, Fill with: Black, Width 400 pixels, Height: 400 pixels, Slices: 1 im Fenster "New Image..."; und
File - Edit - Paste

Im Weiteren wird der Ausschnitt einer Bildbearbeitung unterzogen. Zunächst wird das Bild geschärft und anschließend Helligkeit und Kontrast angepasst. Hierzu werden die Befehle
Process - Sharpen;
Process - Find edges; und
Image - Adjust - Brightness/Contrast
verwendet. In dem Fenster "B&C" werden die Einstellungen "Minimum", "Maximum", "Brightness" und "Contrast" so gewählt, dass die in dem Bildausschnitt zu sehenden an der Schnittfläche offengelegten Partikel weiß dargestellt werden. Das Polymer sowie darin in der Tiefe des Bildes verborgene Partikel werden bei der zu wählenden Einstellung schwarz dargestellt. Somit wird im Wesentlichen ein Zweistufenbild erhalten, in welchem die sich an der Schnittfläche befindenden elektrisch leitfähigen Partikel weiß dargestellt sind. Dies wird mit den Befehlen
Adjust;
Process - Binary - Make binary
umgesetzt. Ferner wird das erhaltene Zweistufenbild negativiert, so dass die Partikel nunmehr schwarz dargestellt werden. Es erfolgt der Befehl
Analyze - Measure Mean.

Die Software zeigt nun ein Fenster "Results" an. Hierin werden die Gesamtfläche des Bildausschnitts unter "Area", der mittlere Grauwert des Bildausschnitts unter "Mean", der minimale Grauwert des Bildausschnitts unter "Min" und der maximale Grauwert des Bildausschnitts unter "Max" angezeigt. Hierbei muss Min = 0 sein, wenn die Bildbearbeitung oben korrekt erfolgt ist. Ferner sollte Max = 255 sein. Der prozentuale Anteil der elektrisch leitfähigen Partikel an der Schnittfläche berechnet sich hieraus zu

$$\text{Partikelanteil [\%]} = (\text{Mean} \cdot 100) / \text{Max}.$$

Der obige Vorgang wird an fünf REM-Aufnahmen der Schnittfläche wiederholt und als Ergebnis für den Partikelanteil in Prozent der arithmetische Mittelwert gebildet. Demnach wird der Anteil der elektrisch leitfähigen Partikel bezogen auf eine Fläche, welche sich in dem zu untersuchenden Abstand von dem Substrat bzw. der Schichtoberfläche befindet angegeben. Soll der Partikelanteil in einem Volumen angegeben werden, werden 3 Schnitte wie oben beschrieben durch dieses Volumen erzeugt und für jeden der Schnitte der Partikelanteil wie oben beschrieben bestimmt. Der Partikelanteil des Volumens ist der arithmetische Mittelwert aus den 3 Schnitten durch das Volumen und wird in Vol.-% angegeben.

Biegsamkeit

Um zu bestimmen, ob ein Substrat oder ein Verbund biegsam ist, wird eine Probe ausreichender Länge des Substrats bzw. des Verbunds verwendet. Ferner sollte die Probe eine Breite von mindestens 1 cm haben. Die Probe wird zu 300° als unvollständige Wicklung um einen Stab mit einem Durchmesser von 20 mm gewickelt. Die Probe wird abgewickelt und in entgegensetzter Richtung wiederum zu 300° um den Stab gewickelt. Die obige Abfolge wird 3 mal wiederholt (insgesamt 6 mal wickeln). Anschließend wird die Probe von Hand auf einem ebenen Untergrund glatt gestrichen. Die Probe wird nun bei 10-facher Vergrößerung unter einem Lichtmikroskop betrachtet. Können hierbei keine Schädigungen der Probenoberfläche gefunden werden, wird der untersuchte Verbund oder das Substrat als biegsam im Sinne der Erfindung angesehen.

spezifische elektrische Leitfähigkeit

Die spezifische elektrische Leitfähigkeit wird als Kehrwert des spezifischen elektrischen Widerstands bestimmt. Der spezifische elektrische Widerstand wird gemäß der Norm ISO 2878:2011(E) bestimmt. Die Messung erfolgt bei einer Temperatur von $23 \pm 2$°C und einer relativen Luftfeuchtigkeit von $50 \pm 5$ %.

Gesamtdicke (Schicht, Beschichtung)

Die Gesamtdicke der Schicht bzw. der Beschichtung wird mit einem Lichtmikroskop mit Skala gemessen. Hierzu wird mit einem Skalpell ein Querschnitt durch den Schichtaufbau erzeugt.

Kontaktwiderstand

Zunächst werden zwei Messkabel mit Hakenklemmen (Fluke AC280 Satz SureGrip) an ein handelsübliches Multimeter angeschlossen. Die Hakenklemmen werden in den folgenden Messungen jeweils mit einem Kontaktdruck von 4 MPa an die Probe angebracht. Zunächst wird die erste Hakenklemme einer ersten Position an die Probe angebracht. Die zweite Klemme wird in einem Abstand $l_1$ an die Probe angebracht. Der elektrische Widerstand $R_1$ wird mit dem Multimeter gemessen. Dann wird die zweite Klemme gelöst und in derselben Richtung wie $l_1$, aber in einem größeren Abstand $l_2$ von der ersten Position an die Probe angebracht. Hierbei sollte unter Berücksichtigung der Probengröße $l_2$ möglichst mindestens doppelt so groß sein wie $l_1$. Nun wird erneut der elektrische Widerstand, jetzt $R_2$, mit dem Multimeter gemessen. Der Kontaktwiderstand $R_{Kontakt}$ berechnet sich zu:

$$R_{Kontakt} = (R_2 l_1 - R_1 l_2)/(2l_1 - 2l_2).$$

Haftfestigkeit

Die Haftfestigkeit wird gemäß der DIN EN ISO 2409:2013-06 bestimmt. Hierbei wird ein Schnittabstand von 60 $\mu$m gewählt und ein Cutter-Messer mit starrer Klinge eingesetzt. Es werden 6 senkrechte Schnitte und, abweichend von der Norm, so viele parallele Schnitte wie möglich, mindestens jedoch 2, durchgeführt. Senkrecht meint hier bei einem längserstreckten Substrat senkrecht zur Richtung der Längserstreckung, bei einem flächenförmigen Substrat senkrecht zur Substratfläche.

Biokompatibilität

Die Biokompatibilität wird gemäß der Norm ISO 10993-4:2002 bestimmt.
Die Erfindung wird im Folgenden durch Beispiele und Zeichnungen genauer dargestellt, wobei die Beispiele und Zeichnungen keine Einschränkung der Erfindung bedeuten.

Vergleichsbeispiel 1 (nicht erfindungsgemäß)

Es werden 500 ml Elastosil A (Wacker Silicones) mit 500 ml Elastisol B (Wacker Silicones) mit 800 ml Carbon-Nano-Tubes (Nanocyl NC 7000 von Safic Alcan) und mit 100 ml Xylene (Diggers) in einem handelsüblichen Küchenmixer gründlich vermischt. Ein Portion des erhaltenen Gemischs wird in eine Spritze mit Öffnungsdurchmesser 1 mm geladen. Auf einen PU-Schlauch mit Durchmesser 150 mm (Vention: Artikel 115-0132) wird von einem Ende zum anderen mit Hilfe der Spritze eine Leiterbahn aus dem Gemisch aufgebracht. Die Leiterbahn auf dem Schlauch wird in einem Umluftofen bei 100 °C für 5 Stunden ausgehärtet.

Vergleichsbeispiel 2 (nicht erfindungsgemäß)

Das Vergleichsbeispiel 2 wird wie das Vergleichsbeispiel 1 durchführt, wobei abweichend 100 ml der Carbon-Nano-Tubes eingesetzt werden.

Beispiel 1 (erfindungsgemäß)

Es werden die drei folgenden Zusammensetzungen vorbereitet.

Zusammensetzung 1:

500 ml Elastosil A (Wacker Silicones)
500 ml Elastisol B (Wacker Silicones)
100 ml Xylene (Diggers)

Zusammensetzung 2:

500 ml Elastosil A (Wacker Silicones)
500 ml Elastisol B (Wacker Silicones)
500 ml Carbon-Nano-Tubes (Nanocyl NC 7000 von Safic Alcan)
100 ml Xylene (Diggers)

Zusammensetzung 3:

500 ml Elastosil A (Wacker Silicones)
500 ml Elastisol B (Wacker Silicones)
800 ml Carbon-Nano-Tubes (Nanocyl NC 7000 von Safic Alcan)
100 ml Xylene (Diggers)

Die oben aufgeführten Komponenten der Zusammensetzung 1 werden für in einem handelsüblichen Küchenmixer gründlich vermischt. Ein Portion jeder gemischten Zusammensetzung 1 wird in eine Spritze mit Öffnungsdurchmesser 1 mm geladen. Auf einen PU- Schlauch mit Durchmesser 150 mm (Vention: Artikel 115-0132) wird von einem Ende zum anderen mit Hilfe der entsprechenden Spritze eine Bahn aus der ersten gemischten Zusammensetzung aufgebracht. Die Bahn auf dem Schlauch wird in einem Umluftofen bei 100 °C für 5 Stunden ausgehärtet. Anschließend werden die Komponenten der Zusammensetzung 2 analog zur oben beschriebenen Vorgehensweise für Zusammensetzung 1 gründlich vermischt, in eine Spritze geladen und die gehärtete Bahn aus der Zusammensetzung 1 auf dem Schlauch mit der gemischten Zusammensetzung 2 überlagert. Die aufgebrachte Zusammensetzung 2 wird wiederum in dem Umluftofen bei 100 °C für 5 Stunden ausgehärtet. Anschließend werden die Komponenten der Zusammensetzung 3 analog zur oben beschriebenen Vorgehensweise für Zusammensetzung 1 gründlich vermischt, in eine Spritze geladen und die gehärtete Bahn aus der Zusammensetzung 2 auf dem Schlauch mit der gemischten Zusammensetzung 3 überlagert. Die aufgebrachte Zusammensetzung 3 wird wiederum in dem Umluftofen bei 100 °C für 5 Stunden ausgehärtet. Es wird ein biegsamer Schlauch mit einer Leiterbahn mit einem erfindungsgemäßen Gradient des Anteils der elektrisch leitfähigen Carbon-Nano-Tubes erhalten.

Auswertung

|  | elektrische Leitfähigkeit | Haftfestigkeit an Substrat |
|---|---|---|
| Vergleichsbeispiel 1 | ++ | -- |
| Beispiel 1 | + | + |
| Vergleichsbeispiel 2 | - | ++ |

In der obigen Tabelle bedeutet ++ ein vorteilhafteres Ergebnis als +, + ein vorteilhafteres Ergebnis als -, und - ein vorteilhafteres Ergebnis als --.
Demnach wird in dem erfindungsgemäßen Beispiel ein biegsamer Schlauch mit einer Leiterbahn erhalten, welche eine vorteilhafte Kombination aus guter elektrischer Leitfähigkeit und guter Haftfestigkeit am Substrat (dem Schlauch) aufweist.
Es zeigen:

Figur 1    eine schematische Querschnittsdarstellung eines erfindungsgemäßen Verbunds;
Figur 2    eine schematische Querschnittsdarstellung eines weiteren erfindungsgemäßen Verbunds;
Figur 3    eine schematische Querschnittsdarstellung eines weiteren erfindungsgemäßen Verbunds;
Figur 4    eine grafische Darstellung des Anteils elektrisch leitfähiger Partikel des Verbunds in Figur 3;
Figur 5    eine schematische Querschnittsdarstellung eines weiteren erfindungsgemäßen Verbunds;
Figur 6    eine schematische Querschnittsdarstellung einer erfindungsgemäßen Vorrichtung mit einer grafischen

EP 3 364 423 B1

Darstellung eines Anteils elektrisch leitfähiger Partikel;

Figur 7          eine schematische Querschnittsdarstellung einer weiteren erfindungsgemäßen Vorrichtung;
Figur 8          ein Flussdiagramm eines erfindungsgemäßen Verfahrens;
Figur 9          ein Flussdiagramm eines weiteren erfindungsgemäßen Verfahrens;
Figur 10         ein Flussdiagramm eines weiteren erfindungsgemäßen Verfahrens;
Figur 11         eine schematische Darstellung eines 3D-Druckers zur erfindungsgemäßen Verwendung;
Figur 12a)       eine schematische Darstellung eines erfindungsgemäßen elektrischen Bauelements;
Figur 12b)       eine schematische Darstellung eines erfindungsgemäßen elektrischen Geräts;
Figur 13         eine schematische Querschnittsdarstellung einer weiteren erfindungsgemäßen Vorrichtung;
Figur 14         eine schematische Darstellung eines Leads mit einem erfindungsgemäßen Verbund;
Figur 15a)       eine schematische Darstellung des Querschnitts A-A in Figur 14;
Figur 15b)       eine schematische Darstellung des Querschnitts B-B in Figur 14;
Figur 15c)       eine schematische Darstellung des Querschnitts C-C in Figur 14; und
Figur 16         eine schematische Darstellung des Längsschnitts D-D in Figur 14.

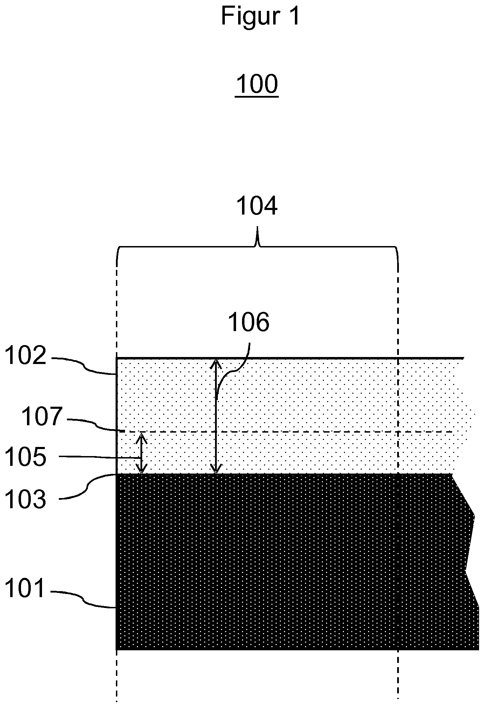

Figur 1 zeigt eine schematische Querschnittsdarstellung eines erfindungsgemäßen Verbunds 100. Der Verbund 100 beinhaltet ein Substrat 101, welches von einer ersten Schicht 102 überlagert wird. Die erste Schicht 102 besteht aus einem Polymer, hier PMMA, und einer Vielzahl elektrisch leitfähiger Partikel, hier einer Vielzahl von Silbernanodrähten. Ferner beinhaltet die erste Schicht 102 eine erste Schichtoberfläche 103, welche direkt an das Substrat 101 angrenzt. In einer ersten Region 104 der ersten Schicht 102 ist die erste Schicht 102 in einem ersten Abstand 105 von 2 μm von der ersten Schichtoberfläche 103 gekennzeichnet durch einen ersten Anteil 403 der elektrisch leitfähigen Partikel von 2 % bezogen auf eine Schnittfläche 107 durch die erste Schicht 102 in dem ersten Abstand 105. Weiterhin in der ersten Region 104 ist die erste Schicht 102 in einem weiteren Abstand 106, welcher gleich einer Dicke der ersten Schicht 102 von 4 μm ist, gekennzeichnet durch einen weiteren Anteil der elektrisch leitfähigen Partikel von 50 % bezogen auf eine Schichtoberfläche der ersten Schicht 102, welche sich in dem weiteren Abstand 106 von dem Substrat 101 befindet. Demnach ist der erste Anteil 403 um 48 % weniger als der weitere Anteil 404 und der erste Abstand 105 ist um 2 μm weniger als der weitere Abstand 106. Das Substrat 101 besteht aus Poly(para-Xylylen), auch als Parylen N bezeichnet.

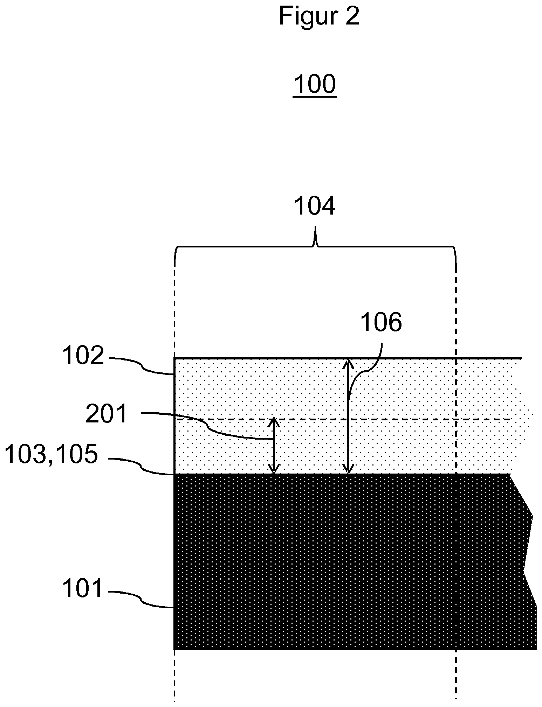

Figur 2 zeigt eine schematische Querschnittsdarstellung eines weiteren erfindungsgemäßen Verbunds 100. Der Verbund 100 nach Figur 2 ist ausgebildet wie der Verbund 100 nach Figur 1, wobei der erste Abstand 105 des Verbunds 100 nach Figur 2 0 μm beträgt und somit an der ersten Schichtoberfläche 103 liegt. Der erste Anteil 403 der elektrisch leitfähigen Partikel beträgt 0 %, bezogen auf die erste Schichtoberfläche 103. Weiter ist die erste Schicht 102 des Verbunds 100 nach Figur 2 in einem zweiten Abstand 201 von 2 μm gekennzeichnet durch einen zweiten Anteil 406 der elektrisch leitfähigen Partikel von 30 Vol.-% bezogen auf eine Schnittfläche durch die erste Schicht 102 in dem zweiten Abstand 201.

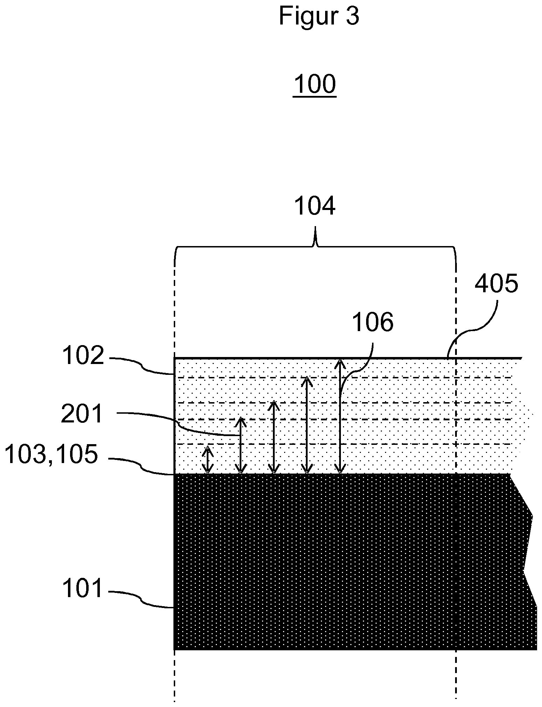

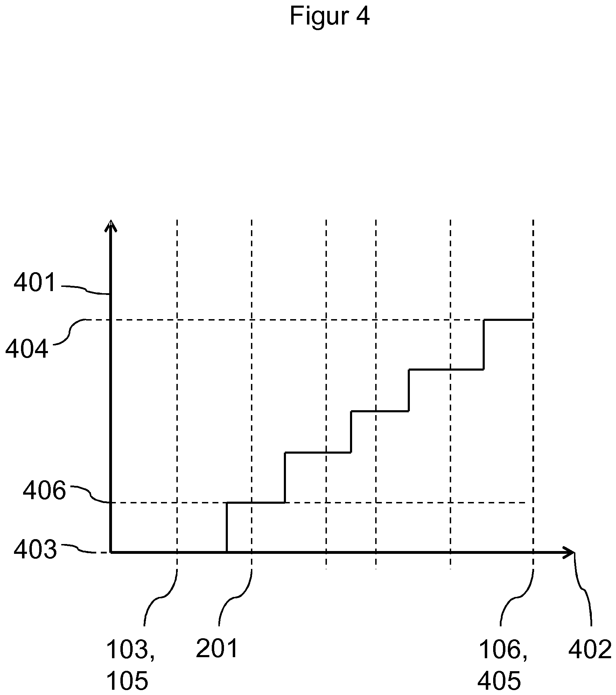

Figur 3 zeigt eine schematische Querschnittsdarstellung eines weiteren erfindungsgemäßen Verbunds 100. Der Verbund 100 nach Figur 3 ist ausgebildet wie der Verbund 100 nach Figur 2, wobei das Polymer PE ist und die elektrisch leitfähigen Partikel Kohlenstoffnanoröhrchen sind. In der ersten Region 104 der ersten Schicht 102 ist die erste Schicht 102 in dem ersten Abstand 105 von 0 μm von der ersten Schichtoberfläche 103 gekennzeichnet durch einen ersten Anteil 403 der elektrisch leitfähigen Partikel von 0 Vol.-% bezogen auf die erste Schichtoberfläche 103. Weiterhin in der ersten Region 104 ist die erste Schicht 102 in dem weiteren Abstand 106, welcher gleich einer Dicke der ersten Schicht 102 von 12 μm ist, gekennzeichnet durch einen weiteren Anteil der elektrisch leitfähigen Partikel von 80 % bezogen auf eine weitere Schichtoberfläche 405. Demnach ist der erste Anteil 403 um 80 % weniger als der weitere Anteil 404 und der erste Abstand 105 ist um 12 μm weniger als der weitere Abstand 106. Weiter ist die erste Schicht 102 des Verbunds 100 nach Figur 3 in dem zweiten Abstand 201 von 2 μm gekennzeichnet durch einen zweiten Anteil 406 der elektrisch leitfähigen Partikel von 30 % bezogen auf eine Schnittfläche durch die erste Schicht 102 in dem zweiten Abstand 201. Ferner ist die erste Schicht 102 des Verbunds 100 nach Figur 3 in einem dritten Abstand von 6 μm gekennzeichnet ist durch einen dritten Anteil der elektrisch leitfähigen Partikel von 40 % bezogen auf eine Schnittfläche durch die erste Schicht 102 in dem dritten Abstand, in einem vierten Abstand von 8 μm gekennzeichnet durch einen vierten Anteil der elektrisch leitfähigen Partikel von 50 % bezogen auf eine Schnittfläche durch die erste Schicht 102 an dem vierten Abstand und in einem fünften Abstand von 10 μm gekennzeichnet ist durch einen fünften Anteil der elektrisch leitfähigen Partikel von 60 % bezogen auf eine Schnittfläche durch die erste Schicht 102 in dem fünften Abstand. In der ersten Region 104 nimmt ein Anteil 401 der elektrisch leitfähigen Partikel von dem ersten Abstand 105, also von der ersten Schichtoberfläche 103, über den zweiten Abstand 201, den dritten Abstand, den vierten Abstand und den fünften Abstand bis zu dem weiteren Abstand 106 zu. Hierbei ist der weitere Abstand 106 gleich einer Dicke der ersten Schicht 102 von 12 μm und liegt somit auf einer der ersten Schichtoberfläche 103 gegenüberliegenden weiteren Schichtoberfläche 405. Die Kohlenstoffnanoröhrchen sind gekennzeichnet durch einen Durchmesser von 12 nm und eine Länge von 30 μm. Einen Graph einer Funktion des Anteils 401 der elektrisch leitfähigen Partikel in der ersten Schicht 102 über einem Abstand 402 von der ersten Schichtoberfläche 103 zeigt Figur 4.

Figur 4 zeigt eine grafische Darstellung des Anteils 401 elektrisch leitfähiger Partikel in der ersten Schicht 102 des Verbunds 100 in Figur 3 über dem Abstand 402 von der ersten Schichtoberfläche 103. Figur 4 zeigt, dass der Anteil 401 der elektrisch leitfähigen Partikel entlang einer Geraden von der ersten Schichtoberfläche 103 zu der weiteren Schichtoberfläche 405 eine monoton steigende Funktion von dem Abstand 402 von der ersten Schichtoberfläche 103 ist. Hier weist die Funktion 5 Stufen auf.

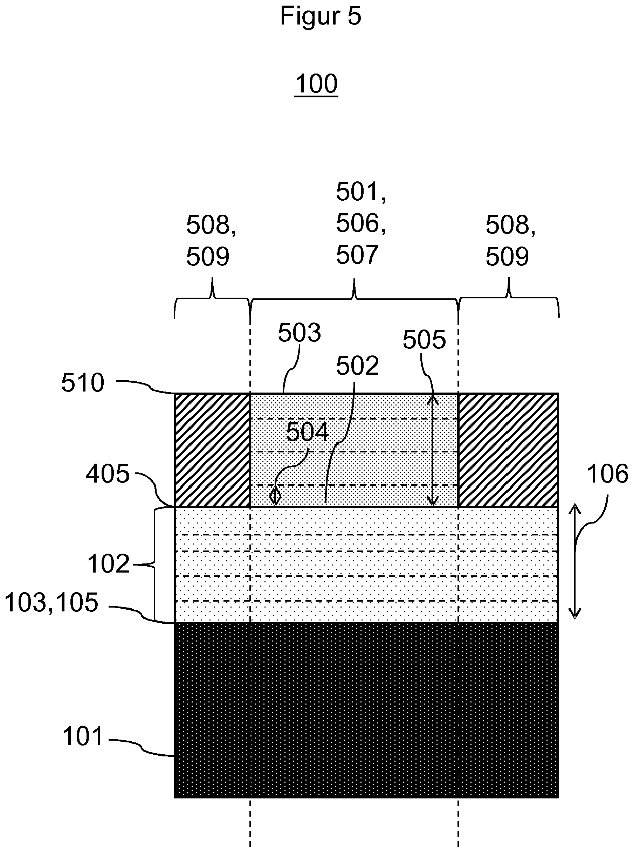

Figur 5 zeigt eine schematische Querschnittsdarstellung eines weiteren erfindungsgemäßen Verbunds 100. Der Verbund 100 nach Figur 5 ist ausgebildet wie der Verbund 100 nach Figur 3, wobei der Verbund 100 weiter eine zusätzliche Schicht 501 beinhaltet. Die zusätzliche Schicht 501 überlagert die erste Schicht 102 in einem ersten Teilbereich 507 der ersten Schicht 102. Hierbei beinhaltet die zusätzliche Schicht 501 eine zusätzliche erste Schichtoberfläche 502, welche an die weitere Schichtoberfläche 405 der ersten Schicht 102 angrenzt. Die zusätzliche Schicht 501 besteht aus dem Polymer, PE, und einer zusätzlichen Vielzahl der elektrisch leitfähigen Partikel, der oben beschriebenen Kohlenstoffnanoröhrchen. In einer weiteren Region 506 der zusätzlichen Schicht 501 ist die zusätzliche Schicht 501 in einem zusätzlichen ersten Abstand 504 von der zusätzlichen ersten Schichtoberfläche 502 gekennzeichnet durch einen zusätzlichen ersten Anteil der elektrisch leitfähigen Partikel von 60 % bezogen auf eine Schnittfläche durch die zusätzliche erste Schicht 501 in dem zusätzlichen ersten Abstand 504. Der zusätzliche erste Abstand 504 beträgt 2 $\mu$m. Ferner ist die zusätzliche Schicht 501 in der weiteren Region 506 in einem zusätzlichen zweiten Abstand von 4 $\mu$m von der zusätzlichen ersten Schichtoberfläche 502 gekennzeichnet durch einen zusätzlichen zweiten Anteil der elektrisch leitfähigen Partikel von 50 % bezogen auf eine Schnittfläche durch die zusätzliche erste Schicht 501 in dem zusätzlichen zweiten Abstand. Weiter ist die zusätzliche Schicht 501 in der weiteren Region 506 in einem zusätzlichen dritten Abstand von 6 $\mu$m von der zusätzlichen ersten Schichtoberfläche 502 gekennzeichnet durch einen zusätzlichen dritten Anteil der elektrisch leitfähigen Partikel von 10 % bezogen auf eine Schnittfläche durch die zusätzliche erste Schicht 501 in dem zusätzlichen dritten Abstand. Zudem ist die zusätzliche Schicht 501 in der weiteren Region 506 in einem zusätzlichen weiteren Abstand 505 von 8 $\mu$m von der zusätzlichen ersten Schichtoberfläche 502 gekennzeichnet durch einen zusätzlichen weiteren Anteil der elektrisch leitfähigen Partikel von 0 % bezogen auf eine Schnittfläche durch die zusätzliche erste Schicht 501 in dem zusätzlichen weiteren Abstand 505. Hier liegt der zusätzliche weitere Abstand 505 an einer zusätzlichen weiteren Schichtoberfläche 503 der zusätzlichen Schicht 501. Die zusätzliche weitere Schichtoberfläche 503 liegt der zusätzlichen ersten Schichtoberfläche 502 gegenüber. Somit ist der zusätzliche weitere Abstand 505 gleich einer Dicke der zusätzlichen Schicht 501. Die zusätzliche weitere Schichtoberfläche ist elektrisch deaktiviert. In einem an den ersten Teilbereich 507 der ersten Schicht 102 angrenzenden weiteren Teilbereich 508 überlagert eine Kontaktierungsschicht 509 die erste Schicht 102 der Art, dass die Kontaktierungsschicht 509 an die weitere Schichtoberfläche 405 angrenzt. Die Kontaktierungsschicht 509 besteht aus dem Polymer und den Kohlenstoffnanoröhrchen, wobei die Kontaktierungsschicht 509 einen Anteil von 90 % bezogen auf das Gesamtvolumen der Kontaktierungsschicht 509 der Kohlenstoffnanoröhrchen aufweist. Somit ist die Kontaktierungsschicht 509 auf einer der ersten Schicht 102 abgewandten Oberfläche 510 der Kontaktierungsschicht 509 gekennzeichnet durch einen gegenüber der zusätzlichen Schicht 501 geringeren Kontaktwiderstand. Die Kontaktierungsschicht 509 besteht aus zwei von einander durch die zusätzliche Schicht 501 getrennten Bereichen, welche beide die weitere Schichtoberfläche 405 der ersten Schicht 102 elektrisch kontaktieren und somit jeweils eine Elektrode zum elektrischen Kontaktieren darstellen.

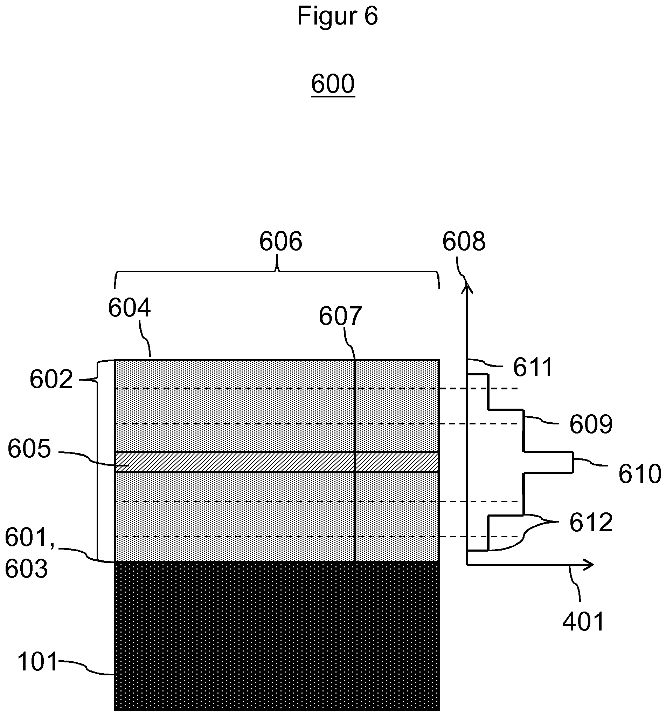

Figur 6 zeigt eine schematische Querschnittsdarstellung einer erfindungsgemäßen Vorrichtung 600 mit einer grafischen Darstellung eines Anteils 401 elektrisch leitfähiger Partikel. Die Vorrichtung 600 beinhaltet ein Substrat 101 und eine Beschichtung 602. Das Substrat 101 beinhaltet eine Substratoberfläche 601 und besteht aus Silikon. Die Beschichtung 602 beinhaltet eine erste Oberfläche 603 sowie eine der ersten Oberfläche 603 gegenüberliegende weitere Oberfläche 604. Hierbei überlagert die Beschichtung 602 das Substrat 101 der Art, dass die erste Oberfläche 603 an die Substratoberfläche 601 angrenzt. Die weitere Oberfläche 604 ist demnach von dem Substrat 101 abgewandt. Die Beschichtung 602 besteht aus einem Polymer und einer Vielzahl elektrisch leitfähiger Partikel. Das Polymer ist PEDOT. Die elektrisch leitfähigen Partikel sind längserstreckte Golddrähten mit einem Durchmesser von 75 nm und einer Länge von 15 $\mu$m. Die Beschichtung 602 ist an der ersten Oberfläche 603 gekennzeichnet durch einen Anteil 401 der elektrisch leitfähigen Partikel von 0 %, bezogen auf die erste Oberfläche 603. Ferner beinhaltet die Beschichtung ein erstes Teilvolumen 605, welches gekennzeichnet ist durch einen Anteil 401 der elektrisch leitfähigen Partikel von 80 Vol.-%, bezogen auf das Volumen der Beschichtung 602 in dem ersten Teilvolumen 605. In einer ersten Region 606 der Beschichtung 602 ist die Beschichtung 602 dadurch gekennzeichnet, dass entlang einer Geraden 607, welche von der ersten Oberfläche 603 zu der weiteren Oberfläche 604 verläuft, ein Anteil 401 der elektrisch leitfähigen Partikel in der Beschichtung 602 eine Funktion 609 von einer Position 608 auf der Geraden 607 mit mindestens einem ersten lokalen Maximum 610 ist. Hierzu zeigt Figur 6 rechts neben der Darstellung der Vorrichtung zur Veranschaulichung ein Diagramm mit einem Graphen der Funktion 609. Das erste lokale Maximum 610 ist von dem ersten Teilvolumen 605 beinhaltet und liegt entsprechend bei 80 Vol.-%. Von dem ersten lokalen Maximum 610 zu jeweils einem in Richtung der ersten Oberfläche 603 und einem in Richtung der weiteren Oberfläche 604 angrenzenden, hier jeweils globalen, Minimum 611 nimmt die Funktion 609 in 3 Stufen 612 ab. Ferner ist die Beschichtung 602 an der weiteren Oberfläche 604 gekennzeichnet durch einen Anteil 401 der elektrisch leitfähigen Partikel von 0 %, bezogen auf die weitere Oberfläche 604. Das erste Teilvolumen 605 ist flächenförmig ausgebildet und erstreckt sich schichtförmig senkrecht zur Bildebene der Figur 6.

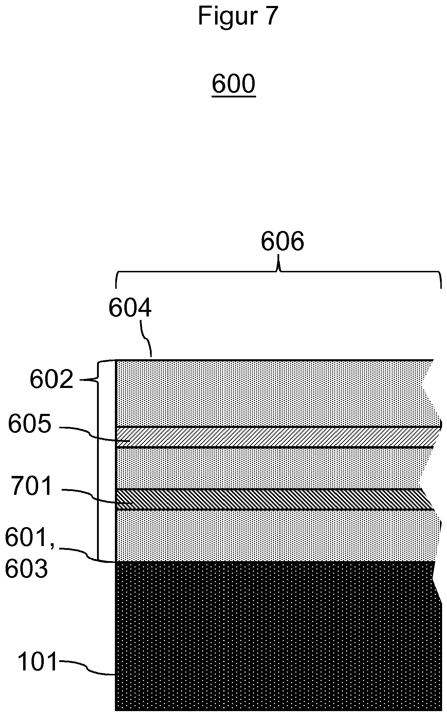

Figur 7 zeigt eine schematische Querschnittsdarstellung einer weiteren erfindungsgemäßen Vorrichtung 600. Die Vorrichtung 600 nach Figur 7 ist ausgebildet wie die Vorrichtung 600 der Figur 6, wobei die Vorrichtung 600 der Figur 7 das erste Teilvolumen 605 und ein weiteres Teilvolumen 701 beinhaltet. In dem ersten Teilvolumen 605 hat die Funktion 609 das erste lokale Maximum 610 bei 0 Vol.-% bezogen auf das Gesamtvolumen des ersten Teilvolumens 605 und in dem weiteren Teilvolumen 701 hat die Funktion 609 ein weiteres lokales Maximum bei 80 Vol.-% bezogen auf das Gesamtvolumen des weiteren Teilvolumens 701. Ferner ist das gesamte weitere Teilvolumen 701 gekennzeichnet durch einen Anteil 401 der elektrisch leitfähigen Partikel in einem Bereich von 80 Vol.-% bezogen auf das Volumen der Beschichtung 602 in dem weiteren Teilvolumen 701. Die Funktion 609 nimmt von dem weiteren lokalen Maximum zu jeweils einem in Richtung der ersten Oberfläche 603 und einem in Richtung der weiteren Oberfläche 604 angrenzenden Minimum 611 in 3 Stufen ab. Somit befindet sich zwischen dem ersten Teilvolumen 605 und dem weiteren Teilvolumen 701 ein Minimum 611 der Funktion 609. In diesem Minimum 611 ist der Anteil 401 der elektrisch leitfähigen Partikel bei 0 % bezogen auf eine Schnittfläche durch die Beschichtung 602. Somit ist das weitere Teilvolumen 701 von dem ersten Teilvolumen 605 elektrisch isoliert. Das weitere Teilvolumen 701 ist analog zum ersten Teilvolumen 605 flächenförmig ausgebildet und erstreckt sich schichtförmig senkrecht zur Bildebene der Figur 7. Das erste Teilvolumen 605 und das weitere Teilvolumen 701 bilden jeweils einen elektrischen Leiter innerhalb der Beschichtung 602. Somit ist die Beschichtung 602 ein zweiphasiger elektrischer Leiter.

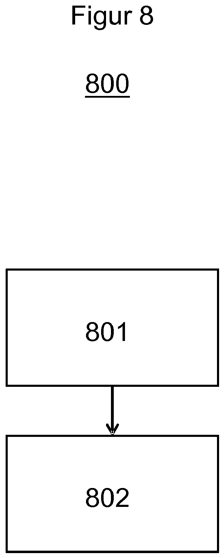

Figur 8 zeigt ein Flussdiagramm eines erfindungsgemäßen Verfahrens 800. Das Verfahren 800 beinhaltet als Verfahrensschritt a) 801 ein Bereitstellen eines Substrats 101 und 3 Zusammensetzungen. Das Substrat 101 beinhaltet wiederum eine Substratoberfläche 601. Jede der 5 Zusammensetzungen beinhaltet ein Polymer, hier PEDOT:PSS, und eine Vielzahl von elektrisch leitfähigen Partikeln, hier Silberplättchen, zu einem Partikelanteil, bezogen auf das Gewicht der jeweiligen Zusammensetzung. Die 3 Zusammensetzungen sind dadurch gekennzeichnet sind, dass sich die Partikelanteile jeweils voneinander unterscheiden. Zusammensetzung 1 hat einen Partikelanteil von 0 Vol.-% bezogen auf das Gesamtvolumen der Zusammensetzung 1. n. Zusammensetzung 2 hat einen Partikelanteil von 30 Vol.-% bezogen auf das Gesamtvolumen der Zusammensetzung 2. n. Zusammensetzung 3 hat einen Partikelanteil von 60 Vol.-% bezogen auf das Gesamtvolumen der Zusammensetzung 3. In einem dem Verfahrensschritt a) 801 nachgelagerten Verfahrensschritt b) 802 wird die Substratoberfläche 601 zunächst in die Zusammensetzung 1 eingetaucht und somit mit einer Portion der Zusammensetzung 1 benetzt. Dann wird diese Portion der Zusammensetzung 1 durch Erwärmen auf 100 °C gehärtet und somit eine erste Schicht, welche die Substratoberfläche 601 überlagert erhalten. Im Weiteren wird eine Oberfläche der ersten Schicht in die Zusammensetzung 2 eingetaucht und somit mit einer Portion der Zusammensetzung 2 benetzt. Dann wird diese Portion der Zusammensetzung 2 wiederum durch Erwärmen auf 100 °C gehärtet und somit eine zweite Schicht, welche die Substratoberfläche 601 und die erste Schicht überlagert erhalten. Weiter wird eine Oberfläche der zweiten Schicht in die Zusammensetzung 3 eingetaucht und somit mit einer Portion der Zusammensetzung 3 benetzt. Dann wird diese Portion der Zusammensetzung 3 wiederum durch Erwärmen auf 100 °C gehärtet und somit eine dritte Schicht, welche die Substratoberfläche 601, die erste Schicht und die zweite Schicht überlagert erhalten. Ferner wird eine Oberfläche der dritten Schicht wiederum in die Zusammensetzung 3 eingetaucht und somit mit einer Portion der Zusammensetzung 3 benetzt. Dann wird diese Portion der Zusammensetzung 3 wiederum durch Erwärmen auf 100 °C gehärtet und somit eine vierte Schicht, welche die Substratoberfläche 601, die erste Schicht, die zweite Schicht und die dritte Schicht überlagert erhalten.

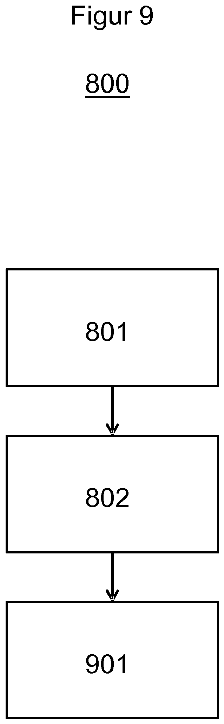

Figur 9 zeigt ein Flussdiagramm eines weiteren erfindungsgemäßen Verfahrens 800. Das Verfahren 800 nach Figur 9 beinhaltet die Verfahrensschritt a) 801 und b) 802 gemäß dem Verfahren 800 der Figur 8 und darüber hinaus einen Verfahrensschritt c) 901. In dem Verfahrensschritt c) 901 wird eine Oberfläche der vierten Schicht in die Zusammensetzung 2 eingetaucht und dadurch mit einer weiteren Portion der Zusammensetzung 2 benetzt. Dann wird diese Portion der Zusammensetzung 2 durch Erwärmen auf 100 °C gehärtet und somit eine zweite Schicht erhalten. Weiter wird eine Oberfläche der weiteren zweiten Schicht in die Zusammensetzung 1 eingetaucht und dadurch mit einer weiteren Portion der Zusammensetzung 1 benetzt. Dann wird diese Portion der Zusammensetzung 1 durch Erwärmen auf 100 °C gehärtet und somit eine weitere erste Schicht erhalten.

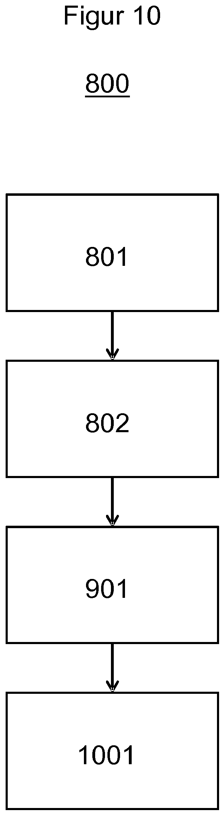

Figur 10 zeigt ein Flussdiagramm eines weiteren erfindungsgemäßen Verfahrens 800. Das Verfahren 800 nach Figur 10 beinhaltet die Verfahrensschritt a) 801, b) 802 und c) 901 gemäß dem Verfahren 800 der Figur 9 und darüber hinaus einen Verfahrensschritt d) 1001. In dem Verfahrensschritt d) 1001 wird eine Oberfläche der weiteren ersten Schicht elektrisch deaktiviert. Dies wird durch partielles Halogenieren der Oberfläche realisiert.

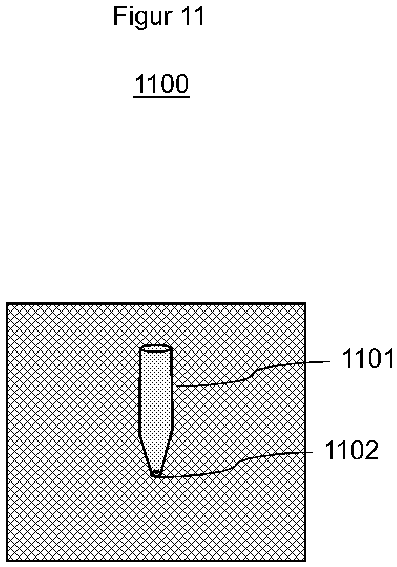

Figur 11 zeigt eine schematische Darstellung eines 3D-Druckers 1100 zur erfindungsgemäßen Verwendung. Der 3D-Drucker 1100 ist ausgebildet zum Erzeugen der Vorrichtung 600 nach Figur 7. Hierfür beinhaltet der 3D-Drucker 1100 eine Düse 1101 mit einer Düsenöffnung 1102 mit einem Durchmesser von 500 nm.

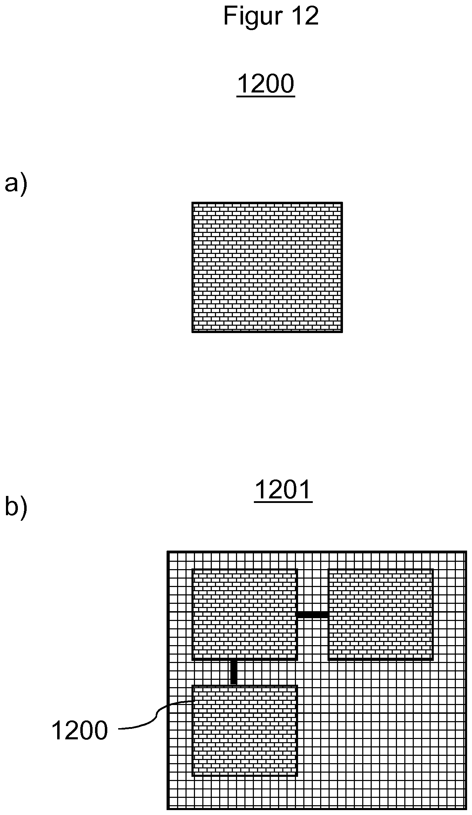

Figur 12a) zeigt eine schematische Darstellung eines erfindungsgemäßen elektrischen Bauelements 1200. Das elektrische Bauelement 1200 ist eine Kapazität beinhaltend die Vorrichtung 600 nach Figur 7.

Figur 12b) zeigt eine schematische Darstellung eines erfindungsgemäßen elektrischen Geräts 1201 beinhaltend 3 erfindungsgemäße elektrische Bauelemente 1200.

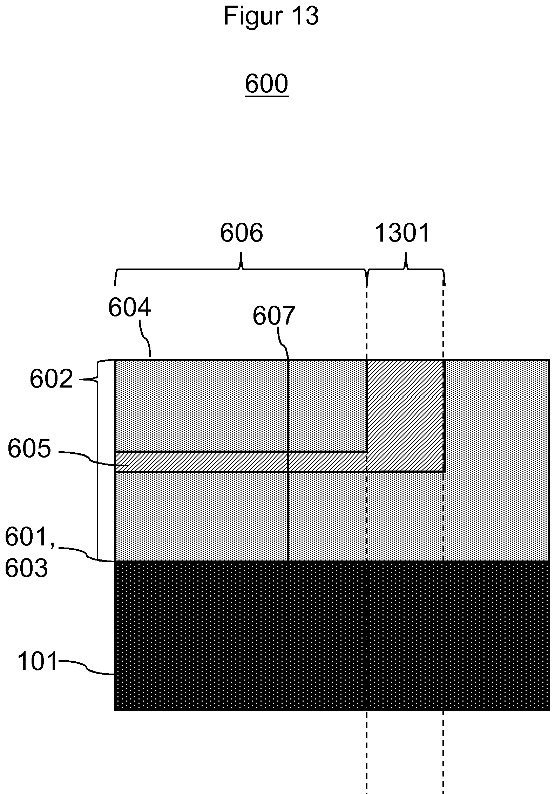

Figur 13 zeigt eine schematische Querschnittsdarstellung einer weiteren erfindungsgemäßen Vorrichtung 600. Die Vorrichtung 600 beinhaltet ein Substrat 101 und eine Beschichtung 602. Das Substrat 101 beinhaltet eine Substratoberfläche 601 und besteht aus Polycarbonat. Die Beschichtung 602 beinhaltet eine erste Oberfläche 603 sowie eine der ersten Oberfläche 603 gegenüberliegende weitere Oberfläche 604. Hierbei überlagert die Beschichtung 602 das Substrat 101 so, dass die erste Oberfläche 603 an die Substratoberfläche 601 angrenzt. Die weitere Oberfläche 604 ist demnach von dem Substrat 101 abgewandt. Die Beschichtung 602 besteht aus einem Polymer und einer Vielzahl elektrisch leitfähiger Partikel. Das Polymer ist SU-8. Die elektrisch leitfähigen Partikel sind Kohlenstoffnanoröhrchen mit einem Durchmesser von 12 nm und einer Länge von 30 μm. Die Beschichtung 602 ist an der ersten Oberfläche 603 gekennzeichnet durch einen Anteil 401 der elektrisch leitfähigen Partikel von 0 %, bezogen auf die erste Oberfläche 603. Ferner beinhaltet die Beschichtung ein erstes Teilvolumen 605, welches gekennzeichnet ist durch einen Anteil 401 der elektrisch leitfähigen Partikel von 80 Vol.-%, bezogen auf das Volumen der Beschichtung 602 in dem ersten Teilvolumen 605. In einer ersten Region 606 der Beschichtung 602 ist die Beschichtung 602 dadurch gekennzeichnet, dass entlang einer Geraden 607, welche von der ersten Oberfläche 603 zu der weiteren Oberfläche 604 verläuft, ein Anteil 401 der elektrisch leitfähigen Partikel in der Beschichtung 602 eine Funktion 609 von einer Position 608 auf der Geraden 607 mit mindestens einem ersten lokalen Maximum 610 ist. Das erste lokale Maximum 610 ist von dem ersten Teilvolumen 605 beinhaltet und liegt entsprechend bei 80 Vol.-%. Von dem ersten lokalen Maximum 610 zu jeweils einem in Richtung der ersten Oberfläche 603 und einem in Richtung der weiteren Oberfläche 604 angrenzenden, hier jeweils globalen, Minimum 611 nimmt die Funktion 609 in 5 Stufen 612 ab. Die Minima 611 liegen hier jeweils an der ersten Oberfläche 603 und der weiteren Oberfläche 604. An der weiteren Oberfläche 604 ist die Beschichtung 602 gekennzeichnet durch einen Anteil 401 der elektrisch leitfähigen Partikel von 0 %, bezogen auf die weitere Oberfläche. In Figur 13 beinhaltet die Beschichtung 602 eine weitere Region 1301, in die sich das erste Teilvolumen 605 erstreckt. In der weiteren Region 1301 beinhaltet das erste Teilvolumen 605 die weitere Oberfläche 604 der Beschichtung 602. Somit kann an der weiteren Oberfläche 604 in der weiteren Region 1301 das erste Teilvolumen 605 elektrisch kontaktiert werden.

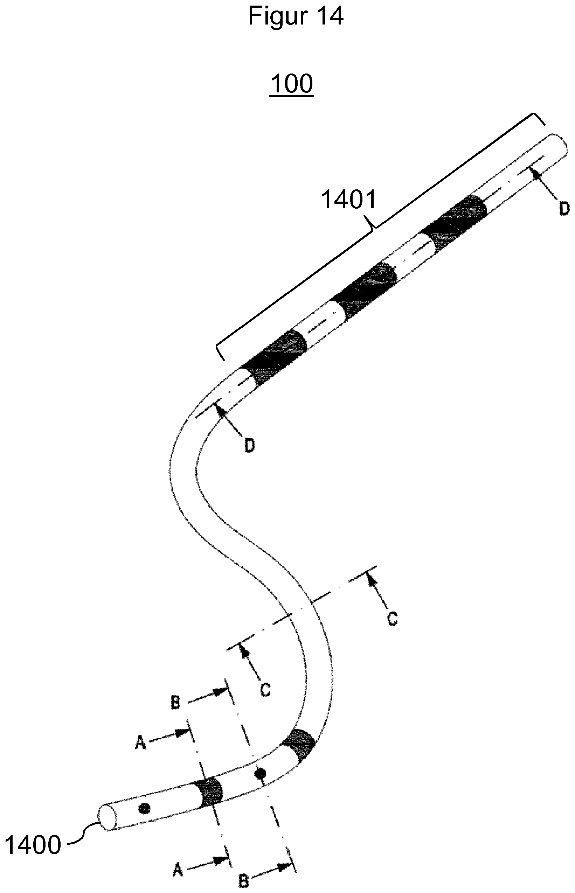

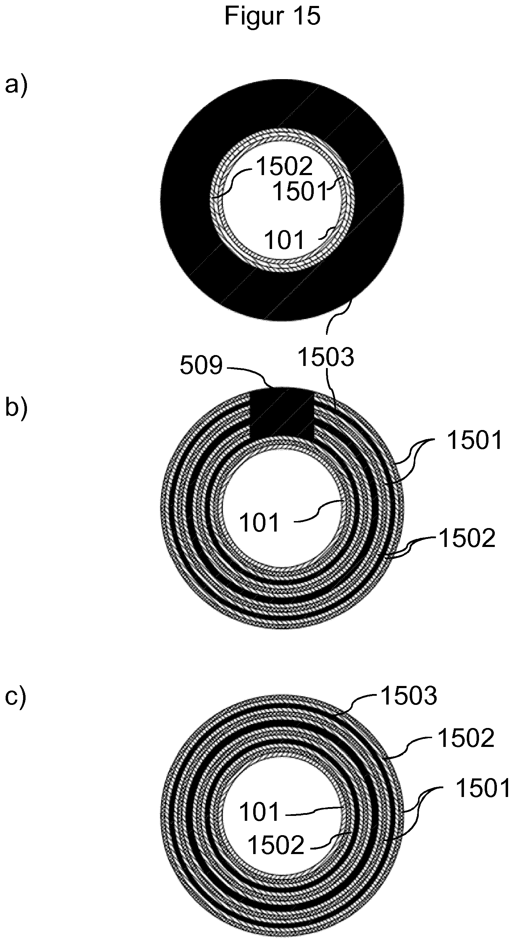

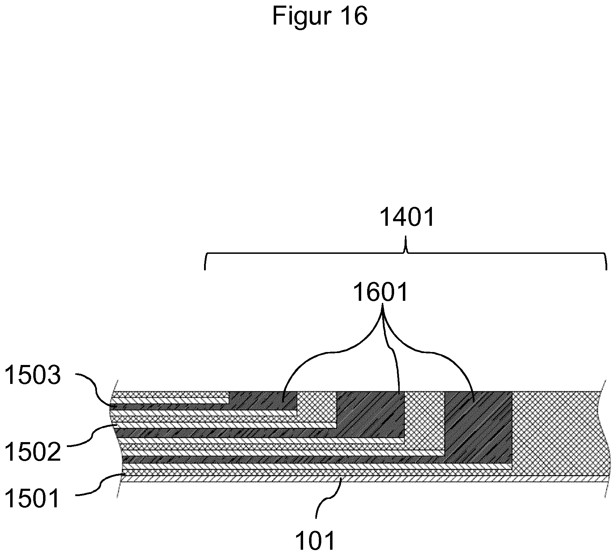

Figur 14 zeigt eine schematische Darstellung eines Leads 1400 mit einem erfindungsgemäßen Verbund 100. Derartige Leads 1400 werden beispielsweise bei implantierbaren Herzschrittmachern als flexibles elektrisches Verbindungselement zwischen dem Impulsgeber und den Elektroden eingesetzt. Hierbei erstreckt sich das Lead von dem Implantierungsort des Herzschrittmachers, häufig unter dem Schüsselbein, bis zu dem zu stimulierenden Herzgewebe. Solche Leads 1400 sind mehrphasige elektrische Leiter, welche biokompatibel, korrosionsbeständig, flexibel, mechanisch beständig und sehr gut elektrisch leitend sein müssen. Hier beinhaltet das Lead 1400 ein Substrat 101 des Verbunds 100. Das Substrat 101 ist mechanisch flexibel und besteht aus Silikon. Ferner sind in der Figur Schnitte A-A, B-B, C-C und D-D angedeutet. Ansichten dieser Schnitte zeigen die Figuren 15a) bis c) sowie Figur 16. Ein Ende des Leads 1400 ist als Stecker 1401 ausgebildet. Über diesen Stecker 1401 kann das Lead 1400 an ein Analysegerät angeschlossen werden und somit Messgrößen wie Druck, Temperatur, Stromstärke oder eine Position des Leads 1400 gemessen und ausgelesen werden.

Figur 15a) zeigt eine schematische Darstellung des Querschnitts A-A in Figur 14. In diesem Querschnitt wird das Substrat 101 überlagert von einer Schichtfolge mit folgenden Schichten 1501, 1502, 1503 in der genannten Reihenfolge: eine Schicht 1501 mit einem Anteil elektrisch leitfähiger Partikel von 0 Vol.-% bezogen auf das Gesamtvolumen der Schicht 1501; eine Schicht 1502 mit einem Anteil elektrisch leitfähiger Partikel von 50 Vol.-% bezogen auf das Gesamtvolumen der Schicht 1502; eine Schicht 1503 mit einem Anteil elektrisch leitfähiger Partikel von 80 Vol.-% bezogen auf das Gesamtvolumen der Schicht 1503. Die Schichten 1501, 1502 und 1503 bestehen jeweils aus einem Polymer und den elektrisch leitfähigen Partikeln jeweils zu den oben genannten Anteilen. Somit bilden die Schichten 1501, 1502 und 1503 gerade eine erste Schicht 102 gemäß dem erfindungsgemäßen Verbund 100. Für alle Schichten 1501, 1502 und 1503 in den Figuren 14 bis 16 ist das Polymer Silikon und die elektrisch leitfähigen Partikel sind Silberplättchen.

Figur 15b) zeigt eine schematische Darstellung des Querschnitts B-B in Figur 14. In diesem Querschnitt wird das Substrat 101 überlagert von einer Schichtfolge mit folgenden Schichten 1501, 1502, 1503 in der genannten Reihenfolge: eine Schicht 1501 mit einem Anteil elektrisch leitfähiger Partikel von 0 Vol.-% bezogen auf das Gesamtvolumen der Schicht 1501; eine Schicht 1502 mit einem Anteil elektrisch leitfähiger Partikel von 50 Vol.-% bezogen auf das Gesamtvolumen der Schicht 1502; eine Schicht 1503 mit einem Anteil elektrisch leitfähiger Partikel von 80 Vol.-% bezogen auf das Gesamtvolumen der Schicht 1503; eine weitere Schicht 1502; eine weitere Schicht 1501; eine weitere Schicht 1502; eine weitere Schicht 1503; eine weitere Schicht 1502, eine weitere Schicht 1501; eine weitere Schicht 1502; eine weitere Schicht 1503; eine weitere Schicht 1502 und eine weitere Schicht 1501. Die Schichten 1501, 1502 und 1503 bestehen jeweils aus einem Polymer und den elektrisch leitfähigen Partikeln jeweils zu den oben genannten Anteilen. Somit bilden die dem Substrat 101 am nächsten liegenden Schichten 1501, 1502 und 1503 gerade eine erste Schicht 102 gemäß dem erfindungsgemäßen Verbund 100. Ferner bilden die auf die erste Schicht 102 als nächste folgenden Schichten 1502 und 1501 eine zusätzliche Schicht 501 gemäß dem erfindungsgemäßen Verbund 100. In einem oberen Bereich des Querschnitts der Figur 15b) ist ein erfindungsgemäßer weiterer Teilbereich 508 zu sehen, in dem die erste Schicht 102 von einer Kontaktierungsschicht 509 überlagert ist. Die Kontaktierungsschicht 509 besteht aus dem Polymer und einem Anteil von 80 Vol.-% der elektrisch leitfähigen Partikel. Somit können die elektrisch leitfähigen Schichten 1503 von außerhalb des Leads 1400 über die Kontaktierungsschicht 509 elektrisch kontaktiert werden. Ferner bilden die Schichten 1501, 1502 und 1503 in Figur 15b) eine erfindungsgemäße Vorrichtung 600 dar. Hierbei bilden alle Schichten 1501, 1502, 1503 eine Beschichtung 602. Die innerste Schicht 1503 bildet ein erstes Teilvolumen 605, die weiten außen liegenden Schichten 1503 bilden jeweils ein weiteres Teilvolumen 701.

Figur 15c) zeigt eine schematische Darstellung des Querschnitts C-C in Figur 14. In diesem Querschnitt wird das Substrat 101 überlagert von einer Schichtfolge mit folgenden Schichten 1501, 1502, 1503 in der genannten Reihenfolge: eine Schicht 1501 mit einem Anteil elektrisch leitfähiger Partikel von 0 Vol.-% bezogen auf das Gesamtvolumen der Schicht 1501; eine Schicht 1502 mit einem Anteil elektrisch leitfähiger Partikel von 50 Vol.-% bezogen auf das Gesamtvolumen der Schicht 1502; eine Schicht 1503 mit einem Anteil elektrisch leitfähiger Partikel von 80 Vol.-% bezogen auf das Gesamtvolumen der Schicht 1503; eine weitere Schicht 1502; eine weitere Schicht 1501; eine weitere Schicht 1502; eine weitere Schicht 1503; eine weitere Schicht 1502, eine weitere Schicht 1501; eine weitere Schicht 1502; eine weitere Schicht 1503; eine weitere Schicht 1502 und eine weitere Schicht 1501. Die Schichten 1501, 1502 und 1503 bestehen jeweils aus einem Polymer und den elektrisch leitfähigen Partikeln jeweils zu den oben genannten Anteilen. Somit bilden die dem Substrat 101 am nächsten liegenden Schichten 1501, 1502 und 1503 gerade eine erste Schicht 102 gemäß dem erfindungsgemäßen Verbund 100. Ferner bilden die auf die erste Schicht 102 als nächste folgenden Schichten 1502 und 1501 eine zusätzliche Schicht 501 gemäß dem erfindungsgemäßen Verbund 100. Ferner bilden die Schichten 1501, 1502 und 1503 in Figur 15c) eine erfindungsgemäße Vorrichtung 600 dar. Hierbei bilden alle Schichten 1501, 1502, 1503 eine Beschichtung 602. Die innerste Schicht 1503 bildet ein erstes Teilvolumen 605, die weiten außen liegenden Schichten 1503 bilden jeweils ein weiteres Teilvolumen 701.

Figur 16 zeigt eine schematische Darstellung des Längsschnitts C-C in Figur 14. Zu sehen ist ein Längsschnitt durch den Stecker 1401 des Leads 1400. Wiederum sind die Schichten 1501, 1502, 1503 dargestellt. Diese sind gerade die in Figur 15c) gezeigten Schichten. Ferner sind die Schichten 1503 jeweils über elektrische Kontakte 1601 des Steckers 1401 elektrisch kontaktierbar.

LISTE DER BEZUGSZEICHEN

| | |
|---|---|
| 100 | erfindungsgemäßer Verbund |
| 101 | Substrat |
| 102 | erste Schicht |
| 103 | erste Schichtoberfläche |
| 104 | erste Region |
| 105 | erster Abstand |
| 106 | weiterer Abstand |
| 107 | Schnittfläche |
| 201 | zweiter Abstand |
| 401 | Anteil elektrisch leitfähiger Partikel |
| 402 | Abstand von der ersten Schichtoberfläche |
| 403 | erster Anteil elektrisch leitfähiger Partikel |
| 404 | weiterer Anteil elektrisch leitfähiger Partikel |
| 405 | weitere Schichtoberfläche |
| 406 | zweiter Anteil elektrisch leitfähiger Partikel |
| 501 | zusätzliche Schicht |
| 502 | zusätzliche erste Schichtoberfläche |
| 503 | zusätzliche weitere Schichtoberfläche |
| 504 | zusätzliche erste Abstand |
| 505 | zusätzliche weitere Abstand |
| 506 | weitere Region |
| 507 | erster Teilbereich |
| 508 | weiterer Teilbereich |
| 509 | Kontaktierungsschicht |
| 510 | Oberfläche der Kontaktierungsschicht |
| 600 | erfindungsgemäße Vorrichtung |
| 601 | Substratoberfläche |

| 602 | Beschichtung |
|---|---|
| 603 | erste Oberfläche |
| 604 | weitere Oberfläche |
| 605 | erstes Teilvolumen |
| 606 | erste Region der Beschichtung |
| 607 | Gerade |
| 608 | Position auf der Geraden |
| 609 | Funktion |
| 610 | erstes lokales Maximum |
| 611 | Minimum |
| 612 | Stufe der Funktion |
| 701 | weiteres Teilvolumen |
| 800 | erfindungsgemäßes Verfahren |
| 801 | Verfahrensschritt a) |
| 802 | Verfahrensschritt b) |
| 901 | Verfahrensschritt c) |
| 1001 | Verfahrensschritt d) |
| 1100 | 3D-Drucker |
| 1101 | Düse |
| 1102 | Düsenöffnung |
| 1200 | erfindungsgemäßes elektrisches Bauelement |
| 1201 | erfindungsgemäßes elektrisches Gerät |
| 1301 | weitere Region der Beschichtung |
| 1400 | Lead |
| 1401 | Stecker |
| 1501 | Schicht mit 0 Vol.-% Anteil elektrisch leitfähiger Partikel |
| 1502 | Schicht mit 40 Vol.-% Anteil elektrisch leitfähiger Partikel |
| 1503 | Schicht mit 80 Vol.-% Anteil elektrisch leitfähiger Partikel |
| 1601 | elektrische Kontakte |

**Patentansprüche**

1. Ein Verbund (100) beinhaltend als einander überlagernde Schichten einer Schichtfolge

    a) ein Substrat (101), und
    b) eine erste Schicht (102);

    wobei die erste Schicht (102)

    i) eine erste Schichtoberfläche (103),
    ii) ein Polymer, und
    iii) eine Vielzahl elektrisch leitfähiger Partikel

    beinhaltet;
    wobei die erste Schichtoberfläche (103) an das Substrat (101) angrenzt;
    wobei mindestens in einer ersten Region (104) die erste Schicht (102) in einem ersten Abstand (105) von der ersten Schichtoberfläche (103) **gekennzeichnet ist durch** einen ersten Anteil (403) der elektrisch leitfähigen Partikel;
    wobei mindestens in der ersten Region (104) die erste Schicht (102) in einem weiteren Abstand (106) von der ersten Schichtoberfläche (103) **gekennzeichnet ist durch** einen weiteren Anteil (404) der elektrisch leitfähigen Partikel;
    wobei der erste Anteil (403) weniger ist als der weitere Anteil (404);
    wobei der erste Abstand (105) weniger ist als der weitere Abstand (106).

2. Der Verbund (100) nach Anspruch 1, wobei mindestens in der ersten Region (104) die erste Schicht (102) in einem zweiten Abstand (201) von der ersten Schichtoberfläche (103) **gekennzeichnet ist durch** einen zweiten Anteil (406) der elektrisch leitfähigen Partikel;
    wobei der zweite Anteil (406) weniger ist als der weitere Anteil (404) und mehr ist als der erste Anteil (403);
    wobei der zweite Abstand (201) weniger ist als der weitere Abstand (106) und mehr ist als der erste Abstand (105).

**3.** Der Verbund (100) nach einem der vorhergehenden Ansprüche, wobei die erste Schicht (102) in der ersten Region (104) **dadurch gekennzeichnet ist, dass** ein Anteil (401) der elektrisch leitfähigen Partikel von dem ersten Abstand (105) zu dem weiteren Abstand (106) zunimmt.

**4.** Der Verbund (100) nach einem der vorhergehenden Ansprüche, wobei die erste Schicht (102) weiter eine der ersten Schichtoberfläche (103) gegenüberliegende weitere Schichtoberfläche (405) beinhaltet, wobei die erste Schicht (102) in der ersten Region (104) **dadurch gekennzeichnet ist, dass** ein Anteil (401) der elektrisch leitfähigen Partikel entlang einer Geraden von der ersten Schichtoberfläche (103) zu der weiteren Schichtoberfläche (405) eine monoton steigende Funktion von einem Abstand (402) von der ersten Schichtoberfläche (103) ist.

**5.** Der Verbund (100) nach einem der vorhergehenden Ansprüche, wobei die erste Schicht (102) an der ersten Schichtoberfläche (103) **gekennzeichnet ist durch** einen Anteil (401) der elektrisch leitfähigen Partikel in einem Bereich von 0 bis 5 %, bezogen auf die erste Schichtoberfläche (103).

**6.** Der Verbund nach einem der vorhergehenden Ansprüche, wobei die elektrisch leitfähigen Partikel aus einem ausgewählt aus der Gruppe bestehend aus Gold, Silber, Palladium, Platin, und Kohlenstoff, oder einer Kombination aus mindestens zwei davon bestehen.

**7.** Der Verbund (100) nach einem der vorhergehenden Ansprüche, wobei das Polymer eines ausgewählt aus der Gruppe bestehend aus Silikon, einem elektrisch leitfähigem Polymer, einem Lack, einem Polyaromaten, einem Thermoplast und einem Harz, oder eine Kombination aus mindestens zwei davon ist.

**8.** Der Verbund (100) nach einem der vorhergehenden Ansprüche, wobei das Substrat (101) aus einem ausgewählt aus der Gruppe bestehend aus einem Kunststoff, einer Kunststoffmischung und einem Metall, oder aus einer Kombination von mindestens zwei davon besteht.

**9.** Der Verbund (100) nach einem der vorhergehenden Ansprüche, wobei das Substrat (101) von einem ausgewählt aus der Gruppe bestehend aus einem medizinischen Gerät, einem medizinischen Hilfsmittel und einem elektrischen Gerät oder von einer Kombination aus mindestens zwei davon beinhaltet ist.

**10.** Der Verbund (100) nach einem der vorhergehenden Ansprüche, wobei das Substrat (101) eines ausgewählt aus der Gruppe bestehend aus einem Schlauch, einem Katheter, einem Draht, einer Nadel, einer Sonde, einem Implantat, einer Folie, einer Kanüle und einem Lead (1400), oder eine Kombination aus mindestens zwei davon ist.

**11.** Der Verbund (100) nach einem der vorhergehenden Ansprüche, wobei der Verbund (100) eines ausgewählt aus der Gruppe bestehend aus einem medizinischen Gerät, einem medizinischen Hilfsmittel, einem Stecker und einer Buchse oder eine Kombination aus mindestens zwei davon ist.

**12.** Eine Vorrichtung (600) beinhaltend ein Substrat (101) und eine Beschichtung (602), wobei das Substrat (101) eine Substratoberfläche (601) beinhaltet; wobei die Beschichtung (602)

a) eine erste Oberfläche (603) und eine weitere Oberfläche (604) beinhaltet, wobei die erste Oberfläche (603) an die Substratoberfläche (601) angrenzt, wobei die weitere Oberfläche (604) von dem Substrat (101) abgewandt ist;
b) ein Polymer und eine Vielzahl elektrisch leitfähiger Partikel beinhaltet;
c) an der ersten Oberfläche (603) **gekennzeichnet ist durch** einen Anteil (401) der elektrisch leitfähigen Partikel in einem Bereich von 0 bis 20 %, bezogen auf die erste Oberfläche (603);
d) ein erstes Teilvolumen (605) beinhaltet; und
e) in dem ersten Teilvolumen (605) **gekennzeichnet ist durch** einen Anteil (401) der elektrisch leitfähigen Partikel in einem Bereich von 1 bis 100 Vol.-%, bezogen auf das Volumen der Beschichtung (602) in dem ersten Teilvolumen (605);

wobei in einer ersten Region (606) der Beschichtung (602)

i) die Beschichtung (602) **dadurch** gekennzeichnet ist, dass entlang einer Geraden (607) von der ersten Oberfläche (603) zu der weiteren Oberfläche (604) ein Anteil (401) der elektrisch leitfähigen Partikel in der Beschichtung (602) eine Funktion (609) von einer Position (608) auf der Geraden (607) mit mindestens einem ersten lokalen Maximum (610) ist,

wobei das erste lokale Maximum (610) von dem ersten Teilvolumen (605) beinhaltet ist,

wobei die Funktion (609) von dem ersten lokalen Maximum (610) zu jeweils einem in Richtung der ersten Oberfläche (603) und einem in Richtung der weiteren Oberfläche (604) angrenzenden Minimum (611) kontinuierlich oder in mindestens 2 Stufen (612) abnimmt; und

ii) die Beschichtung (602) an der weiteren Oberfläche (604) **gekennzeichnet ist durch** einen Anteil (401) der elektrisch leitfähigen Partikel in einem Bereich von 0 bis 20 %, bezogen auf die weitere Oberfläche (604).

**13.** Ein Verfahren (800) beinhaltend als Verfahrensschritte (801, 802)

a) Bereitstellen eines Substrats (101) und n Zusammensetzungen;

wobei das Substrat (101) eine Substratoberfläche (601) beinhaltet,

wobei jede der n Zusammensetzungen ein Polymer und eine Vielzahl von elektrisch leitfähigen Partikeln zu einem Partikelanteil, bezogen auf das Gewicht der jeweiligen Zusammensetzung, beinhaltet,

wobei die n Zusammensetzungen **dadurch gekennzeichnet sind, dass** sich die Partikelanteile der n Zusammensetzungen jeweils voneinander unterscheiden; und

b) Überlagern der Substratoberfläche (601) mit jeweils mindestens einer ersten Portion der n Zusammensetzungen,

wobei das Überlagern mit mindestens den ersten Portionen der n Zusammensetzungen nacheinander erfolgt,

wobei nach jedem Überlagern mit mindestens der ersten Portion einer der n Zusammensetzungen, die mindestens eine erste Portion gehärtet wird,

wobei mindestens die ersten Portionen der n Zusammensetzungen in einer Reihenfolge zunehmender Partikelanteile überlagert werden,

wobei eine erste Schichtfolge beinhaltend als sich einander überlagernde Schichten von der Substratoberfläche (601) in einer Schichtfolgenrichtung eine erste bis n-te Schicht erhalten wird,

wobei n eine natürliche Zahl größer 1 ist

**14.** Eine Vorrichtung erhältlich durch das Verfahren (800) nach Anspruch 13.

**15.** Ein elektrisches Bauelement (1200) beinhaltend den Verbund (100) nach einem der Ansprüche 1 bis 11, oder die Vorrichtung (600) nach einem der Ansprüche 12 oder 14.

**16.** Ein elektrisches Gerät (1201) beinhaltend den Verbund (100) nach einem der Ansprüche 1 bis 11, oder die Vorrichtung (600) nach einem der Ansprüche 12 oder 14, oder das elektrische Bauelement (1200) nach Anspruch 15.

**17.** Eine Verwendung eines 3D-Druckers (1100) zum Erzeugen des Verbunds (100) nach einem der Ansprüche 1 bis 11; oder zum Erzeugen der Vorrichtung (600) nach einem der Ansprüche 12 oder 14.

**18.** Eine Verwendung einer Zusammensetzung, beinhaltend ein Polymer und eine Vielzahl elektrisch leitfähiger Partikel, zu einem elektrischen Kontaktieren einer ein Substrat (101) überlagernden Beschichtung unter Erhalt des Verbunds (100) nach einem der Ansprüche 1 bis 11.

**Claims**

**1.** A composite (100) comprising as superimposed layers of a layer sequence

a) a substrate (101), and
b) a first layer (102);

wherein the first layer (102)

i) a first layer surface (103),
ii) a polymer, and
iii) a multitude of electrically conductive particles

is included;
wherein the first layer surface (103) is adjacent to the substrate (101);
wherein at least in a first region (104) the first layer (102) is **characterized by** a first portion (403) of the electrically conductive particles at a first distance (105) from the first layer surface (103);
wherein at least in the first region (104) the first layer (102) is **characterized by** a further distance (106) from the first layer surface (103) by a further portion (404) of the electrically conductive particles;
wherein the first share (403) is less than the other share (404);
wherein the first distance (105) is less than the further distance (106).

2. The composite (100) according to claim 1, wherein at least in the first region (104) the first layer (102) at a second distance (201) from the first layer surface (103) is **characterized by** a second portion (406) of the electrically conductive particles;
wherein the second share (406) is less than the further share (404) and is more than the first share (403)
wherein the second distance (201) is less than the further distance (106) and is more than the first distance (105).

3. The composite (100) according to any of the foregoing claims, wherein the first layer (102) in the first region (104) is **characterized in that** a proportion (401) of the electrically conductive particles increases from the first distance (105) to the further distance (106).

4. The composite (100) according to any of the preceding claims, wherein the first layer (102) further includes a further layer surface (405) opposite the first layer surface (103), wherein the first layer (102) in the first region (104) is **characterized in that** a portion (401) of the electrically conductive particles along a straight line from the first layer surface (103) to the further layer surface (405) is a monotonically increasing function of a distance (402) from the first layer surface (103).

5. The composite (100) according to any of the foregoing claims, wherein the first layer (102) on the first layer surface (103) is **characterized by** a proportion (401) of the electrically conductive particles in a range of 0 to 5%, based on the first layer surface (103).

6. The composite according to any of the foregoing claims, wherein the electrically conductive particles consist of one selected from the group consisting of gold, silver, palladium, platinum, and carbon, or a combination of at least two thereof.

7. The composite (100) according to any of the foregoing claims, wherein the polymer is one selected from the group consisting of silicone, an electrically conductive polymer, a paint, a polyaromatic, a thermoplastic and a resin, or a combination of at least two of them.

8. The composite (100) according to any of the foregoing claims, wherein the substrate (101) consists of one selected from the group consisting of a plastic, a plastic mixture and a metal, or a combination of at least two thereof.

9. The composite (100) according to any of the foregoing claims, wherein the substrate (101) is comprised of one selected from the group consisting of a medical device, a medical aid and an electrical device or a combination of at least two of them.

10. The composite (100) according to any of the foregoing claims, wherein the substrate (101) is one selected from the group consisting of a tube, catheter, wire, needle, probe, implant, foil, cannula and lead (1400), or a combination of at least two of them.

11. The composite (100) according to any of the foregoing claims, wherein the composite (100) is one selected from the group consisting of a medical device, a medical aid, a plug and a socket, or a combination of at least two thereof.

12. A device (600) comprising a substrate (101) and a coating (602), wherein the substrate (101) comprises a substrate surface (601);
wherein the coating (602)

    a) includes a first surface (603) and another surface (604),
    wherein the first surface (603) is adjacent to the substrate surface (601),
    wherein the further surface (604) is remote from the substrate (101);

b) contains a polymer and a multitude of electrically conductive particles;
c) on the first surface (603) is **characterized by** a proportion (401) of the electrically conductive particles in a range from 0 to 20%, relative to the first surface (603);
d) includes a first partial volume (605); and
e) in the first partial volume (605) is **characterized by** a proportion (401) of the electrically conductive particles in a range from 1 to 100% by volume, based on the volume of the coating (602) in the first partial volume (605);

wherein in a first region (606) of the coating (602)

i) the coating (602) is **characterized in that** along a straight line (607) from the first surface (603) to the further surface (604) a portion (401) of the electrically conductive particles in the coating (602) is a function (609) of a position (608) on the straight line (607) having at least a first local maximum (610),
wherein the first local maximum (610) is contained by the first partial volume (605),
wherein the function (609) decreases continuously or in at least two steps (612) from the first local maximum (610) to one adjacent minimum (611) in the direction of the first surface (603) and one adjacent minimum (611) in the direction of the further surface (604); and
ii) the coating (602) on the further surface (604) is **characterized by** a proportion (401) of the electrically conductive particles in a range from 0 to 20%, relative to the further surface (604).

13. A method (800) comprising as method steps (801, 802)

a) Providing a substrate (101) and n compositions;
wherein the substrate (101) includes a substrate surface (601),
wherein each of the n compositions comprises a polymer and a plurality of electrically conductive particles to a particle fraction based on the weight of the respective composition,
wherein the n compositions are **characterized in that** the particle fractions of the n compositions are different from each other; and
b) superimposing on the substrate surface (601) at least a first portion of each of the n compositions,
the superimposition of at least the first portions of the n compositions being successive,
wherein after each superposition with at least the first portion of one of the n compositions, the at least one first portion is cured,
wherein at least the first portions of the n compositions are superimposed in a sequence of increasing particle fractions,
wherein a first layer sequence including as overlapping layers from the substrate surface (601) in a layer sequence direction a first to n-th layer is obtained,
where n is a natural number greater than 1

14. A device obtainable by the method (800) according to claim 13.

15. An electrical component (1200) comprising the composite (100) according to any of claims 1 to 11, or the device (600) according to any of claims 12 or 14.

16. An electrical apparatus (1201) comprising the composite (100) according to any one of claims 1 to 11, or the device (600) according to any one of claims 12 or 14, or the electrical component (1200) according to claim 15.

17. A use of a 3D printer (1100) for producing the composite (100) according to any one of claims 1 to 11; or for producing the device (600) according to any one of claims 12 or 14.

18. A use of a composition comprising a polymer and a plurality of electrically conductive particles for electrically contacting a coating overlying a substrate (101) to obtain the composite (100) according to any one of Claims 1 to 11.


**Revendications**

1. Composite (100) comprenant comme couches superposées d'une séquence de couches

a) un substrat (101), et
b) une première couche (102) ;

où la première couche (102)

> i) une première couche de surface (103),
> ii) un polymère, et
> iii) une multitude de particules électriquement conductrices

est inclus ;
où la surface de la première couche (103) est adjacente au substrat (101) ;
dans laquelle, au moins dans une première région (104), la première couche (102) est **caractérisée par** une première partie (403) des particules électriquement conductrices à une première distance (105) de la surface de la première couche (103) ;
dans laquelle, au moins dans la première région (104), la première couche (102) est **caractérisée par** une distance supplémentaire (106) de la surface de la première couche (103) par une autre partie (404) des particules électriquement conductrices ;
où la première part (403) est inférieure à l'autre part (404) ;
où la première distance (105) est inférieure à la distance supplémentaire (106).

**2.** Composite (100) selon la revendication 1, dans lequel, au moins dans la première région (104), la première couche (102) à une deuxième distance (201) de la surface de la première couche (103) est **caractérisée par** une deuxième partie (406) des particules électriquement conductrices ;
où la deuxième part (406) est inférieure à l'autre part (404) et supérieure à la première part (403) ;
où la deuxième distance (201) est inférieure à la distance supplémentaire (106) et supérieure à la première distance (105).

**3.** Le composite (100) selon l'une des revendications précédentes, dans lequel la première couche (102) dans la première région (104) est **caractérisée en ce qu'**une proportion (401) des particules électriquement conductrices augmente de la première distance (105) à la distance supplémentaire (106).

**4.** Le composite (100) selon l'une des revendications précédentes, dans lequel la première couche (102) comprend en outre une autre surface de couche (405) opposée à la surface de la première couche (103),
dans laquelle la première couche (102) dans la première région (104) est **caractérisée en ce qu'**une partie (401) des particules électriquement conductrices le long d'une ligne droite de la surface de la première couche (103) à la surface de l'autre couche (405) est une fonction monotone croissante d'une distance (402) de la surface de la première couche (103).

**5.** Le composite (100) selon l'une des revendications précédentes, dans lequel la première couche (102) sur la surface de la première couche (103) est **caractérisée par** une proportion (401) des particules électriquement conductrices dans une plage de 0 à 5%, par rapport à la surface de la première couche (103).

**6.** Composite selon l'une des revendications précédentes, dans lequel les particules électriquement conductrices consistent en une particule choisie dans le groupe constitué par l'or, l'argent, le palladium, le platine et le carbone, ou une combinaison d'au moins deux d'entre elles.

**7.** Le composite (100) selon l'une des revendications précédentes, dans lequel le polymère est un polymère choisi dans le groupe constitué par le silicone, un polymère conducteur de l'électricité, une peinture, un polyaromatique, un thermoplastique et une résine, ou une combinaison d'au moins deux d'entre eux.

**8.** Le composite (100) selon l'une des revendications précédentes, dans lequel le substrat (101) est constitué d'un élément choisi dans le groupe constitué par une matière plastique, un mélange de matières plastiques et un métal, ou une combinaison d'au moins deux de ceux-ci.

**9.** Le composite (100) selon l'une des revendications précédentes, dans lequel le substrat (101) est composé d'un élément choisi dans le groupe constitué par un dispositif médical, une aide médicale et un dispositif électrique ou une combinaison d'au moins deux d'entre eux.

**10.** Le composite (100) selon l'une des revendications précédentes, dans lequel le substrat (101) est un substrat choisi dans le groupe constitué par un tube, un cathéter, un fil, une aiguille, une sonde, un implant, une feuille, une canule et une âme conductrice (in English: a lead) (1400), ou une combinaison d'au moins deux d'entre eux.

**11.** Le composite (100) selon l'une des revendications précédentes, dans lequel le composite (100) est un élément choisi dans le groupe constitué par un dispositif médical, une aide médicale, une fiche et une prise, ou une combinaison d'au moins deux de ces éléments.

**12.** Dispositif (600) comprenant un substrat (101) et un revêtement (602), dans lequel le substrat (101) comprend une surface de substrat (601) ;
où le revêtement (602)

a) comprend une première surface (603) et une autre surface (604),
où la première surface (603) est adjacente à la surface du substrat (601),
où la surface supplémentaire (604) est éloignée du substrat (101) ;
b) contient un polymère et une multitude de particules électriquement conductrices;
c) sur la première surface (603) est **caractérisée par** une proportion (401) de particules électriquement conductrices dans une plage de 0 à 20%, par rapport à la première surface (603) ;
d) comprend un premier volume partiel (605)
e) dans le premier volume partiel (605) est **caractérisée par** une proportion (401) de particules électroconductrices dans une plage de 1 à 100 % en volume, par rapport au volume du revêtement (602) dans le premier volume partiel (605) ;

où dans une première région (606) du revêtement (602)

i) le revêtement (602) est **caractérisé en ce que** le long d'une ligne droite (607) allant de la première surface (603) à l'autre surface (604), une partie (401) des particules électriquement conductrices dans le revêtement (602) est fonction (609) d'une position (608) sur la ligne droite (607) ayant au moins un premier maximum local (610),
où le premier maximum local (610) est contenu par le premier volume partiel (605),
dans lequel la fonction (609) diminue de façon continue ou en au moins deux étapes (612) à partir du premier maximum local (610) jusqu'à un minimum adjacent (611) dans la direction de la première surface (603) et un minimum adjacent (611) dans la direction de l'autre surface (604) ; et
ii) le revêtement (602) sur l'autre surface (604) est **caractérisé par** une proportion (401) de particules électriquement conductrices dans une plage de 0 à 20%, par rapport à l'autre surface (604).

**13.** Une méthode (800) comprenant comme étapes de la méthode (801, 802)

a) Fournir un substrat (101) et n compositions ;
où le substrat (101) comprend une surface de substrat (601),
dans laquelle chacune des n compositions comprend un polymère et une pluralité de particules électriquement conductrices à une fraction de particules basée sur le poids de la composition respective,
où les n compositions sont **caractérisées en ce que** les fractions de particules des n compositions sont différentes les unes des autres
b) en superposant à la surface du substrat (601) au moins une première partie de chacune des n compositions,

la superposition d'au moins les premières parties des n compositions étant successives, dans laquelle, après chaque superposition avec au moins la première partie de l'une des n compositions, la au moins une première partie est durcie,
dans laquelle au moins les premières parties des n compositions sont superposées dans une séquence de fractions de particules croissantes,
dans lequel on obtient une première séquence de couches comprenant comme couches superposées à partir de la surface du substrat (601) dans une direction de séquence de couches une première à n-ième couche,
où n est un nombre naturel supérieur à 1.

**14.** Un dispositif pouvant être obtenu par la méthode (800) selon la revendication 13.

**15.** Composant électrique (1200) comprenant le composite (100) selon l'une des revendications 1 à 11, ou le dispositif (600) selon l'une des revendications 12 ou 14.

**16.** Appareil électrique (1201) comprenant le composite (100) selon l'une quelconque des revendications 1 à 11, ou le dispositif (600) selon l'une quelconque des revendications 12 ou 14, ou le composant électrique (1200) selon la revendication 15.

17. Utilisation d'une imprimante 3D (1100) pour produire le composite (100) selon l'une quelconque des revendications 1 à 11 ; ou pour produire le dispositif (600) selon l'une quelconque des revendications 12 ou 14.

18. Utilisation d'une composition comprenant un polymère et une pluralité de particules électriquement conductrices pour établir un contact électrique avec un revêtement recouvrant un substrat (101) afin d'obtenir le composite (100) selon l'une quelconque des revendications 1 à 11.

Figur 1

100

Figur 2

<u>100</u>

Figur 3

100

# Figur 4

Figur 5

<u>100</u>

# Figur 6

## 600

# Figur 7

## 600

Figur 8

800

801

802

# Figur 9

## 800

801

802

901

# Figur 10

## 800

```
┌─────────────────────┐
│                     │
│         801         │
│                     │
└─────────────────────┘
           │
           ▼
┌─────────────────────┐
│                     │
│         802         │
│                     │
└─────────────────────┘
           │
           ▼
┌─────────────────────┐
│                     │
│         901         │
│                     │
└─────────────────────┘
           │
           ▼
┌─────────────────────┐
│                     │
│        1001         │
│                     │
└─────────────────────┘
```

Figur 11

1100

1101

1102

Figur 12

1200

a)

b)

1201

1200

# Figur 13

## 600

# Figur 14

## 100

1401

1400

# Figur 15

a)

b)

c)

# Figur 16

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2014246220 A1